# EUROPEAN PATENT APPLICATION

(11) **EP 1 787 661 A1**
(43) Date of publication of application: **23.05.2007**
(21) Application number: 05782241.3
(22) Date of filing: 07.09.2005
(51) Int. Cl.: A61K 49/00, A61K 47/26, A61K 47/34

(54) **MEDICINAL PREPARATION**

(30) Priority: 10.09.2004 JP 2004263979; 01.04.2005 JP 2005106146
(71) Applicant: TORAY INDUSTRIES, INC., Tokyo 103-8666 (JP)
(72) Inventor: AOKI, Takao, Naka-gun, Kanagawa 2590132 (JP); YAMANE, Shinichi, 2520805 (JP); KAWANAMI, Shinichi, Kanagawa 2480036 (JP); KOYAMATSU, Yuichi, Kamakura-shi, Kanagawa 2480031 (JP); WATANABE, Yoshifumi, Kanagawa 2210865 (JP); HAMACHI, Itaru, 6170006 (JP); SUZUKI, Ryo, 2291135 (JP); KOIWA, Masakazu, Kanagawa 2210005 (JP); IDA, Nobuo, 2480033 (JP)
(74) Representative: Kador & Partner
(86) International application number: PCT/JP2005/016415
(87) International publication number: WO 2006/028129

(57) **Abstract**

A pharmaceutical preparation has a ligand structure specifically recognizing a target site and an amphiphilic compound having a hydrophobic or amphiphilic group. The pharmaceutical preparation employs an amphiphilic compound of specific structure obtained by introducing a chained hydrophilic group with an appropriate flexibility, and thus becomes a fine particle suited for drug targeting.

The pharmaceutical preparation is expected to give a prolonged pharmacological effect. A particulate preparation exhibiting a remarkable site targeting property can be formed. Further, according to the selection of matrix forming material, the drug releasing property can be controlled.

## Description

### TECHNICAL FIELD

The present invention relates to a pharmaceutical preparation which effectively delivers bioactive substances, drugs, contrast agents, genes, and the like, that is, a pharmaceutical preparation known as a so-called drug delivery system. More specifically, for example, the invention relates to a sustained-release preparation which controls the release of drugs and to a targeting pharmaceutical preparation for delivering drugs to a target tissue.

### BACKGROUND ART

Techniques of directly modifying a drug with a ligand having high affinity for a cell surface material (receptor) in organs and tissues of the site of action, and techniques of supporting a drug on a particulate preparation modified with a ligand, are expected as the technique for giving a targeting function to a drug and are examined for various purposes.

In particular, the carbohydrate recognition mechanism exists *in vivo* is well applied for targeting. For example, it has been known that there exists an organ-specific protein lectin having the binding site for sugar. Since the kind of sugars to be bonded differs with respect to each organ, the ligand having a specific sugar on its end as a ligand for targeting is expected to exhibit the function.

In recent years, there has been a report for the ligand having sugar on its end for such site targeting delivery, that the aggregation state of sugar i.e., forming a cluster (sugar cluster) is important (See Non-Patent Literature 1). Accordingly, a simple technique for forming a cluster of ligands is important for the effective targeting delivery technique. In the clustering technique, for example, considering the ligand for forming a sugar cluster, there has been a problem in the past that its synthesis is extremely complicated because of having a branched structure, or non-biodegradable polymer chain thereof cannot be used in practical.

Meanwhile, there has found a molecule which forms a self-aggregate in water and has properties as a hydrogel, in addition to having a simple structure for easy synthesis. One of the inventors of the present invention, Hamachi et al, found that a glycolipid compound, which has a saccharide as a hydrophilic part and an alkyl chain as a hydrophobic part where therebetween a specific segment exhibiting a hydrogen bonding property is introduced, forms a supramolecular polymer having a nanofiber form in water and forms a hydrogel due to the high molecular aggregability (for example, see Patent Literature 1 and Non-Patent Literature 2). However, the reported compound has a strong molecular aggregability and thus it has been difficult to form particles suitable for drug targeting.
[Patent Literature 1] Japanese Patent Application Laid-Open No. 2000-229992
[Non-Patent Literature 1] Joseph J. Lundquist et al and one other person, Chemical Reviews, vol. 102 (2002), 555-578
[Non-Patent Literature 2] Itaru Hamachi et al and 3 other persons, Journal of the American Chemical Society, vol. 124(2002), 10954-10955

### [DISCLOSURE OF THE INVENTION]

The first invention of the present invention is a pharmaceutical preparation containing an amphiphilic compound represented by the following general formula (I): 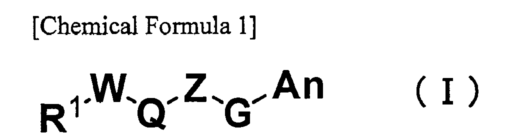 (wherein R¹ is a ligand structure which specifically recognizes a target site; W is a group which includes any one of -NH-, -O-, and -S-; Q is a chained hydrophilic group; Z is -O-, -S-, or -NR² (where R² is hydrogen, a methyl group, an ethyl group, a normal propyl group, an isopropyl group, an acetyl group, a benzyl group, a hydroxy group, or a methoxy group); G is a group represented by the following general formula (II): 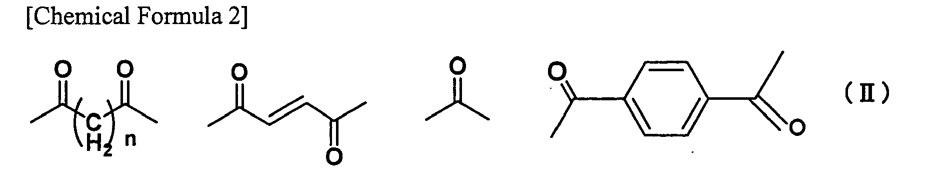 (where, n is an integer of 0 to 9); and An is a hydrophobic or amphiphilic group).

The other invention of the present invention is a pharmaceutical preparation comprising a hydrophobic supermagnetic metal oxide, a biodegradable polymer, an amphiphilic compound, and particles having an average particle size of 25 to 300 nm.

### [BRIEF DESCRIPTION OF THE DRAWINGS]

Fig. 1 shows a structure of an amphiphilic compound (VII).
Fig. 2 shows a structure of an amphiphilic compound (VIII).
Fig. 3 shows a structure of an amphiphilic compound (IX).
Fig. 4 shows a structure of an amphiphilic compound (X).
Fig. 5 shows a structure of an amphiphilic compound (XI).
Fig. 6 shows a structure of an amphiphilic compound (XII).
Fig. 7 shows a structure of an amphiphilic compound (XIII).
Fig. 8 shows a synthesis scheme up to Compound (5), in a synthesis scheme of the amphiphilic compound (VII).
Fig. 9 shows a synthesis scheme starting from the Compound (5), in a synthesis scheme of the amphiphilic compound (VII).
Fig. 10 shows a synthesis scheme up to Compound (7), in a synthesis scheme of the amphiphilic compound (VIII).
Fig. 11 shows a synthesis scheme starting from the Compound (7), in a synthesis scheme of the amphiphilic compound (VIII).
Fig. 12 shows a synthesis scheme up to Compound (10), in a synthesis scheme of the amphiphilic compound (X).
Fig. 13 shows a synthesis scheme starting from the Compound (10), in a synthesis scheme of the amphiphilic compound (X).
Fig. 14 shows a synthesis scheme up to Compound (15), in a synthesis scheme of the amphiphilic compound (XI).
Fig. 15 shows a synthesis scheme starting from the Compound (15), in a synthesis scheme of the amphiphilic compound (XI).
Fig. 16 shows a synthesis scheme of the amphiphilic compound (XII).
Fig. 17 shows a synthesis scheme up to Compound (18), in a synthesis scheme of the amphiphilic compound (XIII).
Fig. 18 shows a synthesis scheme starting from the Compound (18), in a synthesis scheme of the amphiphilic compound (XIII).
Fig. 19 is a result obtained by evaluating the uptake of a fluorescent magnetite-enclosed particulate preparation prepared by the amphiphilic compounds (VIII), (X), (XII), (XIII), by hepatic parenchymal cells.

### BEST MODE FOR CARRYING OUT THE INVENTION

The pharmaceutical preparation of the invention is a pharmaceutical preparation containing an amphiphilic compound which has a targeting part for specifically recognizing a target site.

An object of the invention is to realize a particulate pharmaceutical preparation which is hardly taken up by the reticuloendothelial system and well retained in blood, and further to provide a pharmaceutical preparation by a simple technique, which is expected to give a remarkable effect on targeting organs such as liver and comprises particles of which the surface is formed with a cluster of ligands (e.g., sugar).

The present inventors have focused on that the basic structure of the low molecular compound forming a hydrogel discovered by Hamachi *et al* has no complicated branched chains or the like and thus is a compound easy to be synthesized, and that the high aggregability due to a hydrogen bond and a hydrophobic bond of the molecule thereof is useful for forming a cluster of ligands such as sugars. Accordingly, extensive studies have been carried out on a compound having such mentioned properties and constituting a particle suitable for drug targeting. As a result, it is found that the particle can be formed by employing an amphiphilic compound, for which the aggregability between the molecules is increased by appropriately arranging groups with hydrogen bonding property in a molecular structure and the specific structure is obtained by introducing a chained hydrophilic group with an appropriate flexibility, with the compound alone or with a combination of a matrix forming material.

First, the first invention of the present invention will be explained. The first invention of the present invention is a pharmaceutical preparation containing the amphiphilic compound represented by the following general formula (I): 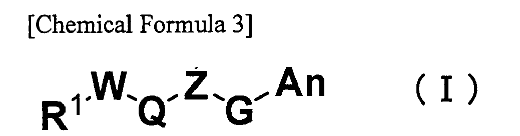 (wherein R¹ is a ligand structure which specifically recognizes a target site; W is a group which includes any one of -NH-, -O-, and -S-; Q is a chained hydrophilic group; Z is -O-, -S-, or -NR² (where R² is hydrogen, a methyl group, an ethyl group, a normal propyl group, an isopropyl group, an acetyl group, a benzyl group, a hydroxy group, or a methoxy group); G is a group represented by the following general formula (II): 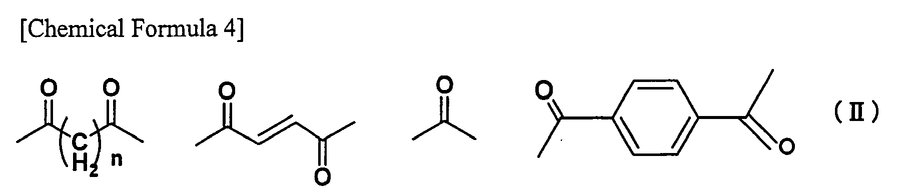 (where, n is an integer of 0 to 9); and An is a hydrophobic or amphiphilic group).

The amphiphilic compound according to the present invention is an amphiphilic compound having a structure represented by the following general formula (I): 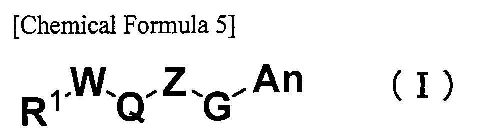 and basically constituted by a targeting part R¹ which specifically recognizes a target site, an anchoring moiety An which comprises a lipophilic group, and a linking moiety -W-Q-Z-G- which links the R¹ and An.

In the invention, the R¹ in the general formula (I) corresponds to a targeting part, and which provides the essential structure for exhibiting affinity for the receptor exists in a target site. The chemical structure of the R¹ is not particularly limited as long as it is a structure specifically recognizing a target site *in vivo.* Specific examples of the R¹ include amino acid, oligopeptide, polypeptide, antibodies, an part of antibody, receptors, enzymes; monosaccharides such as glucose, mannose, mannose-6-phosphoric acid, galactose, glucosamine, lactosamine, galactosamine, N-acetylglucosamine, N-acetylgalactosamine, gluconic acid, glucuronic acid, and galacturonic acid, and aldonic acid derivatives thereof; disaccharides such as lactose, maltose, melibiose, cellobiose, isomaltose, and sucrose, and aldonic acid derivatives thereof; oligosaccharides such as trehalose, sialyl Lewis X, and sialyl Lewis alpha, and aldonic acid derivatives thereof; polysaccharides such as dextran, pullulan, mannan, heparin, low molecular weight heparin, hyaluronic acid, dermatan sulfate, chondroitin sulfate, keratan sulfate, and syndecan, and derivatives thereof; and the like, but may not be limited by those.

For the R¹ in the general formula (I), the R¹ in the general formula (I) is preferably a monosaccharide and/or a derivative thereof, disaccharide and/or a derivative thereof, oligosaccharide and/or a derivative thereof, or polysaccharide and/or a derivative thereof.

For the R¹ in the general formula (I), the R¹ in the general formula (I) is preferably a monosaccharide, and may be galactose, N-acetylgalactosamine, mannose, glucose, N-acetylglucosamine, maltose, or the aldonic acid derivative thereof.

For the R¹ in the general formula (I), the R¹ in the general formula (I) is preferably a disaccharide or oligosaccharide, and its end thereof is galactose, N-acetylgalactosamine, mannose, glucose, N-acetylglucosamine, or a maltose group.

For the R¹ in the general formula (I), the R¹ in the general formula (I) is preferably a disaccharide, and may be lactose, cellobiose, gentibiose, melibiose, or the aldonic acid derivative thereof.

In addition, the R¹ in the general formula (I) is preferably trisaccharide 2'-fucosyllactose, tetrasaccharide 2',3-difucosyllactose, trisaccharide 2,3-difucosyllactose, or the aldonic acid derivative thereof.

The R¹ in the general formula (I) is preferably a Lewis X-type trisaccharide chain, a sialyl Lewis X-type tetrasaccharide chain, a 3'-sialyl lactosamine trisaccharide chain, a 6'-sialyl lactosamine trisaccharide chain, or the aldonic acid derivative thereof.

The R¹ in the general formula (I) is preferably amino acid, oligopeptide, polypeptide, an antibody, an part of antibody, a receptor, an enzyme, folic acid, porphyrin, oligonucleic acid, and/or a derivative thereof.

In particular, in case of using the amphiphilic compound to provide affinity for the liver or hepatic parenchymal cells, R¹ is preferably galactose; galactosamine; N-acetylgalactosamine; mannose; a disaccharide, oligosaccharide, polysaccharide, or the aldonic acid derivative thereof, of which the end is galactose or galactosamine, N-acetylgalactosamine, or mannose; or apo B. The saccharide chain may be branched. The saccharide chain is preferably the ones already having galactose or galactosamine, N-acetylgalactosamine, or mannose on the end of each branched chain or the part thereof. In addition, it is preferably the saccharide chain giving galactose or galactosamine, N-acetylgalactosamine, or mannose, on the saccharide end by the metabolic mechanism *in vivo.*

In case of using the amphiphilic compound to provide affinity for the macrophage, R¹ is preferably mannose, galactosamine, or an oligosaccharide or polysaccharide having mannose or galactosamine on its end. The saccharide chain may be branched. The saccharide chain is preferably the ones already having mannose or galactosamine on the end of each branched chain or the part thereof. In addition, it is preferably the saccharide chain giving mannose or galactosamine, on the saccharide end by the metabolic mechanism *in vivo.* In addition, as the oligopeptide, tuftsin can be preferably used.

In particular, in case of using the amphiphilic compound to provide affinity for the T cell, R¹ is preferably gp120g derived from HIV virus.

Further, in case of using the amphiphilic compound to provide affinity for cancer cells or tumor tissues, the R¹ is preferably a ligand which binds to various molecules present in blood vessels of cancer cells or tumor tissues, for example, a folic acid receptor, a transferrin receptor, various growth factors (EGF, VEGF, FGF, PDGF, etc.), various growth factor receptors, various hormone receptors, adhesion molecules, chemokine receptors (CCR6, CCR7, etc.), and various tumor markers. As the ligand, proteins such as an antibody; peptides; sugars; nucleic acids; derivatives thereof; or various low molecular synthetic compounds can be preferably used.

In the invention, An in the general formula (I) corresponds to an anchoring moiety. The An in the general formula (I) provides the essential structure for exhibiting affinity for drugs or matrix forming materials included in a pharmaceutical preparation. The An in the general formula (I) is not particularly limited as long as it is a hydrophobic or amphiphilic group.

The An in the general formula (I) is preferably a hydrophobic or amphiphilic group having the structure specifically represented by the following formula (III) or (IV): 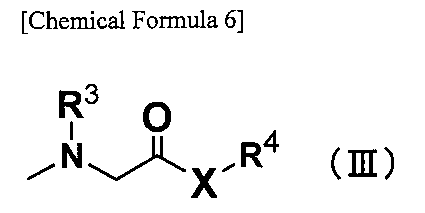 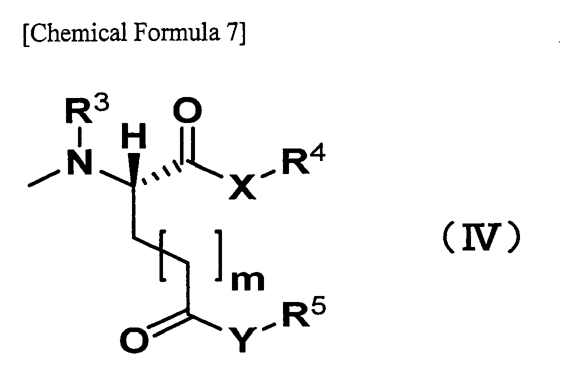 (wherein R³ is hydrogen, a methyl group, an ethyl group, a normal propyl group, an isopropyl group, an acetyl group, a benzyl group, a hydroxy group, or a methoxy group; X and Y are each independently or same -NR⁶-, -O-, or -S-, (where R⁶ is hydrogen or an alkyl group having 1 to 20 carbon atom(s)); and m is an integer of 0 to 4). The R⁶ is preferably a hydrophobic or amphiphilic group represented by a straight-chained alkyl group having 1 to 20 carbon atom(s), a branched alkyl group having 1 to 20 carbon atom(s), a straight-chained alkyl group having 2 to 20 carbon atoms which contains a double bond, a branched alkyl group having 2 to 20 carbon atoms which contains a double bond, or -CH₂R⁷ (where R⁷ is an aryl group or a cycloalkyl group having 3 to 8 carbon atoms).

R⁴ and R⁵ are preferably a hydrophobic or amphiphilic group represented by a straight-chained alkyl group having 1 to 20 carbon atom(s), a branched alkyl group having 1 to 20 carbon atom(s), a straight-chained alkyl group having 2 to 20 carbon atoms which contains a double bond, a branched alkyl group having 2 to 20 carbon atoms which contains a double bond, or -CH₂R⁷ (where R⁷ is an aryl group or a cycloalkyl group having 3 to 8 carbon atoms). R⁴ and R⁵ are most preferably have 6 to 20 carbon atoms and further preferably in a chained structure, by the reasons of high affinity for a matrix forming material and excellent orientation of the molecules adjacent to each other. When R⁴ and R⁵ are each -CH₂-R⁷, the R⁷ is preferably an aryl group or a cycloalkyl group having 3 to 8 carbon atoms, by the same reason. In addition, when R⁴ and R⁵ are each an alkenyl group, 1 to 5 double bond(s) is/are preferably included by the reason of high affinity for a matrix forming material.

In the invention, the linking moiety refers to all structures included between the targeting part R¹ and anchoring moiety represented as An in the general formula (I), and the aggregation state of the amphiphilic compound on the surface of particles can be varied according to the property of the linking moiety. In the amphiphilic compound, a substituent having a hydrogen bonding property is preferably placed on an appropriate position in the linking moiety for the purpose of forming an aggregation of molecules.

In the invention, the linking moiety W is a group which includes any one of -NH-, -O-, and -S-.

Specifically, the W is preferably -O-, -NH-, or -S-, and particularly preferably is -NH- from the point of hydrogen bonding property.

The W is preferably a group represented by the following general formula (VI):

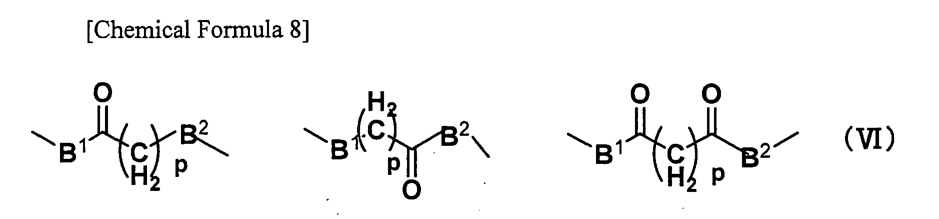

While, specific examples of the B¹ and B², which may be independent or same with each other, preferably include -O-, -NH-, and -S-, and particularly preferably include -NH- from the point of hydrogen bonding property. In addition, p is an integer of 0 to 9, and particularly preferably is an integer of 1 to 4.

In the invention, Q in the general formula (I) is a chained hydrophilic group. Specific examples of the Q include polyethyleneglycol, polyethyleneimine, polyethylene oxide, peptide, nucleic acid, and their derivatives, but may not be limited by those.

In particular, the Q is preferably a hydrophilic group represented by the following general formula (V):

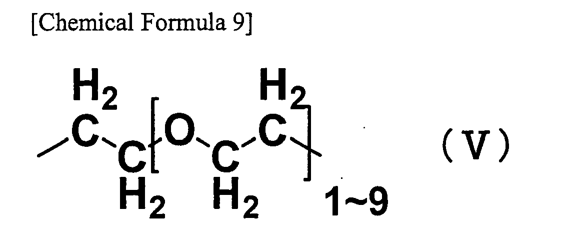

In the invention, Z in the general formula (I) is -O-, -NR²-, or -S-. R² is hydrogen, a methyl group, an ethyl group, a normal propyl group, an isopropyl group, an acetyl group, a benzyl group, a hydroxy group, or a methoxy group. Z is preferably -NR²- particularly from the point of hydrogen bonding property. In addition, R² is preferably any one of hydrogen, a hydroxy group, and an acetyl group, from the point of hydrogen bonding property.

In the invention, G in the general formula (I) is a group represented by the formula (II), and n is an integer of 0 to 9. For the group represented by the formula (II), n is preferably an integer of 2 to 4.

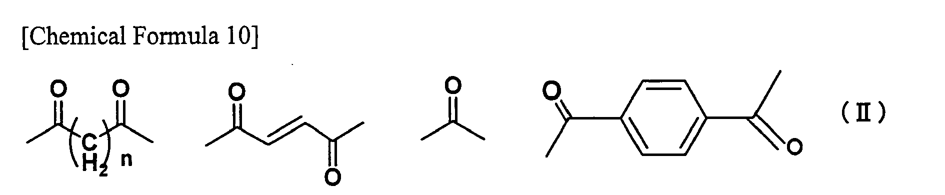

In the invention, a method of bonding R¹ and W in the general formula (I) is not particularly limited and the method can be appropriately selected.

As the R¹, when a saccharide having the reducing terminal, which is typified by a monosaccharide, disaccharide, oligosaccharide, and polysaccharide, is bonded with any one of ether bond, thioether bond, and amino bond, the hydroxy group of the saccharide is in general protected by an acyl group, a haloacyl group, a benzylidene group, or an isopropylidene group. The protection reaction is described in detail in "Sugar Science and Engineering" (Kodansha) written by Hatanaka *et al.* Considering the facility of protection and deprotection, an acetyl group or a haloacetyl group is preferably used. The protected sugar is dissolved in a solvent such as chloroform or chloroethane, and subjected to the reaction by adding W of having any of hydroxy group, thiol group, and amino group in the presence of a Lewis acid catalyst such as tin tetrachloride and trifluorinated boron. Thereafter, the catalyst and the solvent are removed, thus obtained product is treated with a Lewis base such as sodium methoxide to remove the protecting group, and thus R¹-W bond is obtained. In the glycosylated compound obtained according to such method, the sugar is bonded with carbon on the anomeric position of reducing end. In addition, the same glycosylated compound can also be obtained by reacting with W in the presence of silver perchlorate instead of the Lewis acid catalyst, after brominating carbon on the anomeric position with hydrogen bromide.

The amphiphilic compound used in the invention can be produced according to an arbitrary method. As shown in the general formula (I), the amphiphilic compound used in the invention has a structure in which the individual molecular part, except the bonding part between R¹ and W, is bonded with any of ester bond, amide bond, thioester bond, and the like. Accordingly, the production can be made by providing one end of the molecular part as an active derivative electrophilic agent of carboxylic acid and the other end of the molecular part as a nucleophilic agent such as alcohol, amine, and thiol body, to a condensation reaction.

As the R¹, when using an aldonic acid derivative of monosaccarides, disaccharides, oligosaccarides, and polysaccharides, the sugar is dissolved in an appropriate solvent such as methanol, refluxed under heating in the co-presence of dehydrating agent to obtain a lactone intermediate, the intermediate is reacted with the nucleophilic agent W, and the solvent is removed to obtain the bonding, or otherwise can also be obtained by first deriving the R¹ to the mentioned active carbonic acid electrophilic agent and then reacting with the nucleophilic agent W. In this case, when there is a hydroxyl group, a thiol group, or an amino group other than the reaction center in a sugar residue, the target R¹-W bond can be obtained by first protecting with an appropriate protecting group such as a benzyl group and then subjecting to the reaction.

The amphiphilic compound used in the invention, the producing solvent, the reaction temperature, the reaction period, and the purification method are appropriately selected in dependence upon the chemical structures of the starting material and precursor material, and the kind of reaction.

In the process for producing the amphiphilic compound used in the invention, the active derivative electrophilic agent of carboxylic acid can be mentioned by carboxylic acid chloride, carboxylic acid bromide, carboxylic acid iodide, Weinreb amide, carboxylic ester, carboxylic anhydride, or carboxylic salt. These can be obtained by activating carboxylic acid with carbodiimides such as dicyclohexylcarbodiimide, then adding the corresponding nucleophilic agent, and removing the urea compound such as dicyclohexyl urea which is produced as a by-product and thereafter removing the solvent, or otherwise can also be obtained according to a process including activating with carbodiimide, then further activating a carboxyl group with N-hydroxy succinimide, and adding a nucleophilic agent.

In addition, the compound can be produced by adding a nucleophilic agent to any of carboxylic acid chloride, carboxylic acid bromide, carboxylic acid iodide, and carboxylic anhydride in the presence of a catalyst such as pyridine, triethylamine, and dimethylaminopyridine, and then removing the solvent after first removing the salt of the catalyst. The compound can also be produced by reacting any of carboxylic acid chloride, carboxylic acid bromide, carboxylic acid iodide, and carboxylic anhydride, with O-methylhydroxylamine to convert into Weinreb amide, then subjecting to a reaction with a nucleophilic agent, and thereafter removing the solvent. Further, the compound can also be obtained by adding a nucleophilic agent to carboxylic ester and then removing the solvent.

The present invention is a pharmaceutical preparation containing the amphiphilic compound represented by the general formula (I).

The amphiphilic compound represented by the general formula (I) can alone enclose a drug or a contrast substance therein. Alternatively, the amphiphilic compound represented by the general formula (I) can enclose a drug or a contrast substance therein in combination with a matrix forming material described later.

In addition, to the pharmaceutical preparation of the invention, a pharmaceutically acceptable base such as a stabilizer, antioxidant, solubilization agent, surfactant, sorbefacient, pH regulator, dispersant, and the like may also be blended in addition to the matrix forming material, the drug, and the contrast substance.

The pharmaceutical preparation of the invention is preferably a particulate preparation constituted by fine particles having an average particle size of 50 µm or less. In the case of using as injectable form, the pharmaceutical preparation is preferably a fine particle constituted by particles having an average particle size of 10 to 300 nm. In particular, for the purpose of being circulated through a blood vessel and taken up by the liver, hepatic parenchymal cells, and tumors, the average particle size is preferably in the range of 50 to 200 nm. For measuring the average particle size of particles directly, the laser scattering particle size analyzer (e.g., Microtrac ASVR/Microtrac-HRA (9320-X100)) can be used.

The method of forming fine particles with the amphiphilic compound used in the invention is not particularly limited as long as the amphiphilic compound used in the invention is used, and can be carried out according to a conventionally known method. Although not limited, methods described below can be employed in general.

The first method comprises preliminarily using the amphiphilic compound to be dissolved in a solvent, next forming precipitates or dispersions from the solvent and non-solvent, and thereafter vaporizing and removing the solvent to collect the particle in the form of a colloidal dispersion. The solvent solution is generally an organic solution of the amphiphilic compound, and the non-solvent solution is preferably aqueous solution, or alcohol solution.

In a case where a water-miscible organic solvent is used as the solvent, if the solution is mixed with water phase, the amphiphilic compound which is insoluble in the water phase, that is the complex of the amphiphilic compound which is insoluble in the water phase/organic solvent mixture, gradually precipitates in the form of a particle. When a non-water miscible organic solvent is used as the solvent, the organic solvent showing no water miscibility in which the amphiphilic compound and the drug are included is emulsified in a water phase, and then the organic solvent is vaporized to be removed.

The second method comprises dissolving the amphiphilic compound used in the invention to a solvent, and preparing an aqueous dispersion of liposome in accordance with a well known method (Ann. Rev. Biophys. Bioeng., 9,467(1980)). The taken liposome may include any of sterols such as cholesterol as a membrane stabilizer, charged substances such as dialkylphosphoric acid and stearylamine, and antioxidant such as tocopherol.

The third method comprises using the amphiphilic compound to be dissolved in a solvent, then mixing the solution with a non-solvent, subjecting the mixture to an ultrasonication, and vaporizing and removing the solvent to collect the particle in the form of a colloidal dispersion. The solvent solution is generally an organic solution of the amphiphilic compound, and the non-solvent solution is often an aqueous solution. When a non-water miscible organic solvent is used as the solvent, the organic solvent showing no water miscibility in which the amphiphilic compound is included is emulsified in a water phase by ultrasonication, and then the organic solvent is vaporized to be removed. In a case where a water-miscible organic solvent is used as the solvent, if the solution is mixed with water phase, the lipid derivative which is insoluble in the water phase, that is the amphiphilic compound which is insoluble in the water phase/organic solvent mixture, gradually precipitates in the form of a particle. The mixture is emulsified by ultrasonication, and then the organic solvent is vaporized to be removed.

In the invention, among the methods described above, it is preferable to employ the method comprising dissolving the amphiphilic compound in a solvent, next forming precipitates or dispersions from the solvent and non-solvent, and thereafter vaporizing and removing the solvent to collect the particle in the form of a colloidal dispersion.

In the invention, the matrix forming material is either involved in forming a particle in addition to the amphiphilic compound used in the invention and the drug, or is a material providing various properties to the drug of the invention. For example, when the matrix forming material is a biodegradable polymer, a function of controlling a retention time for a target site of the drug can be provided to the preparation, and when the matrix forming material is any of oils and fats and perfluorocarbon, the same function of controlling a retention time for a target site can also be provided. This is also same in the case where the matrix forming material is a liposome forming material. In addition, the kind of the drug which can be included in a particle can be selected by the selection of each matrix forming material.

In the invention, the matrix forming material is preferably a biodegradable polymer. The biodegradable polymer is capable of controlling the retention time for a target site, and it is low in accumulation and development of toxicity caused by the accumulation. The biodegradable polymer of the invention is a high molecular weight compound which can be metabolized or degraded generally in the external or *in vivo* environment, without needing a specific manipulation. The biodegradable polymer of the matrix forming material is a polymer having a property of not exhibiting a defect on organisms when after administered *in vivo* and then fit in the living tissue, for example a polymer having a property of being degraded and metabolized *in vivo* and finally excreted outside the body. The biodegradable polymer is not particularly limited in the structure, but a polymer which is poorly soluble or non-soluble in water is generally used. Specific examples of the biodegradable polymer can be mentioned by the followings.

### 1. Aliphatic polyesters:

(1) Homopolymers, copolymers, which is synthesized from one or more of alpha-hydroxy carboxylic acids (such as glycolic acid, lactic acid, 2-hydroxybutyric acid, 2-hydroxy valeric acid, 2-hydroxy caproic acid, 2-hydroxy capric acid), hydroxy dicarboxylic acids (such as malic acid), hydroxy tricarboxylic acids (such as citric acid), and the like, or mixtures thereof.
(2) Homopolymers, copolymers, which is synthesized from one or more of lactides (such as glycolide, lactide, benzylmalolactonate, malide benzyl ester, 3-[(benzyloxycarbonyl)methyl]-1,4-dioxane-2,5-dione), and the like, or mixtures thereof.
(3) Homopolymers, copolymers, which is synthesized from one or more of lactones (such as beta-propiolactone, delta-valerolactone, epsilon- caprolactone, N-benzyloxycarbonyl-L-serine-beta-lactone), and the like, or mixtures thereof. These can be copolymerized with glycolides, lactides, etc. as cyclic dimers of alpha-hydroxy acids.

### 2. Polyanhydrides:

For example, poly[1,3-bis(p-carboxyphenoxy)methane], poly(terephthalic-sebacic anhydride, and the like.

### 3. Polycarbonates:

For example, poly(oxycarbonyloxyethylene), spiro-ortho-polycarbonate, and the like.

### 4. Polyorthoesters:

For example, poly{3,9-bis(ethylidene-2,4,8,10-tetraoxaspiro[5,5]undecane-1,6-hexanediol)}, and the like.

### 5. Poly-alpha-cyanoacrylic acid esters:

For example, isobutyl poly-alpha-cyanoacrylate, and the like.

### 6. Polyphosphazenes:

For example, polydiaminophosphazene, and the like.

### 7. Polypeptides or polyamino acids, and derivatives thereof.

These biodegradable polymers may be used as a mixture in appropriate ratios. The type of polymerization of the biodegradable polymer may be any of random, block, and graft polymerization.

Among the above-mentioned biodegradable polymers, preferably used examples include aliphatic polyesters [such as polymers, copolymers, which is synthesized from one or more of alpha-hydroxycarboxylic acids (such as glycolic acid, lactic acid, hydroxybutyric acid), hydroxydicarboxylic acids (e.g., malic acid, etc.), hydroxytricarboxylic acids (such as citric acid,), and the like, or mixtures thereof, or polylactides]. In particular, homopolymers or copolymers synthesized from one or more of alpha-hydroxy carboxylic acids (such as glycolic acid, lactic acid, hydroxybutyric acid), or polylactides are preferably used from the viewpoint of biocompatibility and biodegradability. These copolymers may be used as a mixture in appropriate ratios.

The alpha-hydroxycarboxylic acids and the polylactides may be any of the D-, L- or D,L-configuration. The alpha-hydroxycarboxylic acids and the polylactides are preferably the ones in which the D-/L-ratio (mole/mole%) falls within the range from about 75/25 to about 25/75. The alpha-hydroxycarboxylic acids and the polylactides with a D-/L-ratio (mole/mole%) within the range from particularly about 60/40 to about 30/70 are widely used. Examples of the copolymers of alpha-hydroxycarboxylic acids include copolymers of glycolic acid with other alpha-hydroxycarboxylic acids, and the alpha-hydroxycarboxylic acids for a use can be exemplified by lactic acid, 2-hydroxybutyric acid, or the like. Specifically, examples of the alpha-hydroxy carboxylic acids preferably include a lactic acid-glycolic acid copolymer, a 2-hydroxybutyric acid-glycolic acid copolymer, and the like, and particularly the lactic acid-glycolic acid copolymer (hereinafter, also may be referred to as lactic acid-glycolic acid, which refers to both a homopolymer and a copolymer of lactic acid, glycolic acid, unless otherwise specified) can be widely used.

The proportion of the lactic acid-glycolic acid copolymer (lactic acid/glycolic acid) (mol/mol%) is not particularly limited as long as the purpose of the invention is achieved, but preferably the one in the range of about 100/0 to about 30/70. The number average molecular weight of the lactic acid-glycolic acid copolymer and polylactide is preferably about 500 to 100,000, and more preferably about 1,000 to 50,000.

The method of forming a particulate preparation using a biodegradable polymer as a matrix forming material is not particularly limited except that the amphiphilic compound used in the invention and the drug are used, and may be performed in accordance with the well known method without being particularly limited. The preferred method is a method which comprises preliminarily using and dissolving the amphiphilic compound, the drug, and the matrix forming material in a solvent, next forming precipitates or dispersions from the solution of polymers and non-solvent, and thereafter vaporizing and removing the solvent to collect the particle in the form of a colloidal dispersion. The solvent solution is generally an organic solution of the polymer, and its non-solvent solution is often an aqueous solution.

In a case where a water-miscible organic solvent is used as the solvent, if the solution is mixed with water phase, the polymer insoluble in the water phase, that is the polymer insoluble in the water phase/organic solvent mixture, gradually precipitates in the form of a particle.

When a non-water miscible organic solvent is used as the solvent, the organic solvent showing no water miscibility in which the polymer is included is emulsified in a water phase, and then the organic solvent is vaporized to be removed.

As the matrix forming material used in the invention, a liposome forming material is preferable. The liposome forming material is preferably used by the reasons of being capable of controlling the retention time for a target site, and low in accumulation and development of toxicity caused by the accumulation. When the matrix forming material is a liposome forming material, although being not particularly limited if it is the amphiphilic substance having the property of forming liposome, examples include lipids such as phosphatidylcholine, sphingomyelin, and phosphatidylethanolamine, membrane component substances such as a dialkyl synthetic surfactant, and the like.

The preparation of the liposome according to the invention is not particular limited except that the amphiphilic compound used in the invention is used, and may be carried out in accordance with the well known method. Basically, the amphiphilic compound used in the invention is dissolved or dispersed in a solvent together with other membrane component to be mixed. Concretely, lipid such as phosphatidylcholine, sphingomyelin, and phosphatidylethanolamine, or a membrane component substance such as an alkyl synthetic surfactant agent, is first mixed with the phospholipid used in the invention, and the aqueous dispersion of liposome is prepared in accordance with the well known method (Ann. Rev. Biophys. Bioeng., 9,467(1980)). The taken liposome may include any of sterols such as cholesterol as a membrane stabilizer, charged substances such as dialkylphosphoric acid and stearylamine, and antioxidant such as tocopherol.

In the liposome prepared in the above manner, the proportion of the amphiphilic compound used in the invention to the total lipid membrane component is preferably about 1/40 molar ratio or more, and more preferably 1/20 molar ratio or more, by reason that a stable particle formation is achieved.

Further, in the invention, most broad of lipids and lipoids can be used alone or in a mixture as the oils and fats of the matrix forming material. In particular, examples of the lipid microspheres and solid lipid nanospheres, as the pharmaceutical particulate preparation, include natural and synthetic triglyceride or arbitrary mixtures thereof, mono- and diglyceride (singularly or in arbitrary mixtures thereof, such as a mixture of triglyceride), natural and synthetic wax, fatty alcohols (including their esters and ethers), lipid peptides, and the like. Particularly preferred examples include individual or mixtures of synthetic mono-, di-, and triglycerides (such as hard fat), glycerin trifatty acid ester (such as glycerin trilaurate, glycerin myristate, triglycerin palmitate, triglycerin stearate and triglycerin behenate), wax [such as cetyl palmitate and white wax (bleaching wax, DAB9)], and the like.

Further, in the invention, the perfluorocarbon of the matrix forming material is not intended to be particularly limited, but partially or entirely fluorinated alkyl, alcohol, alkylether, and the like can be included for a use. Preferred examples include perfluorodecalin, perfluorooctane, perfluorodichlorooctane, perfluoroheptane, perfluorodecane, perfluorocyclohexane, perfluoromorpholine, perfluorotripropylamine, perfluorotributylamine, perfluorodimethylcyclohexane, perfluorotrimethylcyclohexane, perfluoro-n-octylbromide, perfluorodicyclohexyl ether, perfluoro-n-butyltetrahydrofuran, and the like.

The process for producing the pharmaceutical preparation of the invention is not particularly limited except that the amphiphilic compound used in the invention is used, and may be carried out in accordance with the well known method without being particularly limited, which includes well known techniques for forming a lipid microspheres or solid lipid nanoparticles. The solid lipid nanoparticles, for example, can be produced according to a process for producing a particulate preparation containing fine particles of lipid-form which is solid at room temperature, prototype lipid (lipoid)-form, or their mixture-form, where the process comprises homogeneously dispersing the inner phase (lipid and/or lipoid) in a molten or soften state in a dispersant (water, aqueous solution, or water miscible liquid) under high pressure, or homogeneously dispersing the inner phase in a finely-powdered solid state in a dispersant under high pressure.

The pharmaceutical preparation of the invention can have various morphologies. The morphology of the invention may include a powder form comprising a particulate preparation and a pharmaceutically acceptable additive; a liquid form comprising any of the mixture of a particulate preparation and a medium such as water and the mixture of polysaccharides, a medium such as water, and a pharmaceutically acceptable base other than the medium; and a solidified or semisolidified form obtained by combining a particulate preparation with a pharmaceutically acceptable base. In addition, the pharmaceutical preparation may be solidified as a lyophilized formulation and then may be turned into liquid at the time of administration by adding a medium.

The pharmaceutically acceptable base can be mentioned by various organic or inorganic substances which are commonly used as a formulation material, and examples include excipient, lubricant, binder, disintegrant, solvent, solubilization agent, suspending agent, isotonizing agent, buffer, soothing agent, sorbefacient, and the like.

As the dispersant, water, an aqueous solution, or a water miscible liquid, such as glycerin or polyethylene glycol is used. The aqueous solution may be any of non-isotonic and isotonic solution. As the aqueous solution, water and one or a plurality of other components, for example, ones prepared by mixing polyoles such as glycerin, mannose, gluocol, fructose, xylose, trehalose, mannite, sorbite, xylite, and polyethylene glycol, and/or an electrolyte such as sodium chloride, can be exemplified. The amount of the component used is 0.1 to 50%, and preferably 1 to 30%, based on a basic composition.

In the invention, the form of the pharmaceutical preparation is not particularly limited. In addition, the size of the pharmaceutical preparation is not particularly limited, and may be suitably selected according to the purpose. The size of the cellular interval may differ by a tissue, and also the size for incorporating cells may vary by a cell. If the size is too large, it becomes hard to be incorporated in tissues or cells, and if the size is too small, it becomes easy to be taken up by other than the targeted tissues or cells. In order to adsorb the particle on a surface of the tissues or cells, the size is preferably in the range of not being taken up by tissues or cells.

The particulate preparation of the pharmaceutical preparation according to the invention preferably has the average particle size of 50 µm or less. For the purpose of being incorporated in tissues or cells, the size is preferably 5 µm or less. In particular, for the purpose of being circulated through a blood vessel and taken up by the liver or hepatic parenchymal cells, the average particle size is preferably in the range of 50 to 200 nm. For the average particle size of particles, the laser scattering particle size analyzer (e.g., Microtrac ASVR/Microtrac-HRA (9320-X100)) can be used to directly determine the average particle size.

The pharmaceutical preparation of the invention is generally blended with a drug (medicinal properties) to be used as a pharmaceutical preparation. The drug is not particularly limited, and can be used in a wide range such as ones used in clinical practice, or ones expected for clinical use can be used.

In the particulate preparation, medicinal agents i.e., one or more of bioactive substance, drug, diagnostic drug, and genes, may be included.

Examples of the bioactive substance include growth factors such as PDGF, VEGF, HGF, FGF, and EGF; cytokines such as INF, TGF, and interleukin; hormones; and the like.

In addition, antigens useful as vaccine such as killed bacterium, toxin, sugar chain, peptides, and the like, are also included. Further, the substance can be exemplified by contrast agent, anticancer drug, antiphlogistic, antiallergic drug, circulatory drug, antihypertensive agent, hypertensive agent, antiplatelet drug, anticoagulant, psychotropic drug, pain killer, antibiotics, digestant, urologic drug, endocrine drug, fluorescent reagent, or the like.

As the gene, oligonucleotide, polynucleotide, DNA, and RNA can be mentioned. Particularly, in the case of targeting a liver or hepatic cells, antiviral agents such as ribavirin, lamivudine, and interferon; antiinflammatory agents such as ciclosporin; anticancer agents such as cisplatin, carboplatin, 5-Fu, mitomycin, cychlophosphamide, methotrexate, and irinotecan hydrochloride; antihyperlipemic drugs such as fibrates; growth factors such as HGF; angiogenesis inhibitors such as endostatin and angiostatin; and liver disease remedy such as a liver protecting agent, can be preferably used.

When the target site is tumor tissues or tumor cells, anticancer agents such as cisplatin, carboplatin, 5-Fu, mitomycin, cychlophosphamide, methotrexate, irinotecan hydrochloride, doxorubicin hydrochloride, and paclitaxel, can be preferably used.

As the diagnostic drug, contrast agents such as magnetite, iopamidol, iohexol, 131I, 99mTc, 131I-HSA, 67Ga, 3H, 24Na, 86Rb, 87mSr, and 18F-FDG, can be used.

In the contrast agent, particularly the MRI contrast material promotes relaxation of hydrogen nucleus (proton) (shorten a relaxation time) in a magnetic resonance phenomenon, thus is a material having the property to increase the contrast on MRI image, which is preferably commonly used supermagnetic metal oxide or paramagnetic metal complex.

The supermagnetic metal oxide useful in the pharmaceutical preparation of the invention is not particularly limited as long as it is the transition metal oxide and is in the range of exhibiting super paramagnetic property. For example, the iron oxide useful in the pharmaceutical preparation of the invention can be exemplified by the ferrite represented by the following general formula:

(MO)m • Fe₂O₃

(wherein, M is a divalent metal atom, and m is a number of 0 ≤ m ≤ 1). Examples of the divalent metal atom include magnesium, calcium, manganese, iron, nickel, cobalt, copper, zinc, strontium, barium, and the like. In particular, magnetic iron oxide for which the M is iron having a valence of 2 (such as magnetite Fe₃0₄, γ-Fe₂0₃) can be preferably used in the invention. The magnetic iron oxide particles in the invention also include the ones with crystal water.

The paramagnetic metal complex useful in the pharmaceutical preparation of the invention is not particularly limited as long as it has paramagnetic property and forms a stable complex. For example, transition metals with atomic number 21 to 29, 42, and 44, and ion complexes having a valence of 2 and 3 of lanthanide series (lanthanaid series) metals with atomic number 58 to 70, can be used. Of these, particularly preferred ones include complexes of chromium, manganese, iron, copper, and gadolinium, and having strong paramagnetic property.

The administration mode of the pharmaceutical preparation is not particularly limited in the invention, but administration by intravenous injection or infusion is preferable.

Next, the second invention of the present invention will be explained.

The second invention of the present invention is a pharmaceutical preparation having hydrophobic supermagnetic metal oxide, a biodegradable polymer, an amphiphilic compound, and a particle which has an average particle size of 25 to 300 nm.

The second invention of the invention is formed with hydrophobic supermagnetic metal oxide, a biodegradable polymer, and an amphiphilic compound.

The amphiphilic compound according to the second invention of the present invention comprises at least two or more parts, in which at least one or more part(s) is/are a hydrophilic part and also at least 1 or more part(s) is/are a hydrophobic part.

Herein, the term 'hydrophilic' is when the water solubility of an arbitrary part is higher than the other segment, the part is referred to as 'hydrophilic'. The hydrophilic part is desirably soluble in water, and a poor solubility is also acceptable as long as the water solubility is higher than the other parts. On the other hand, the term 'hydrophobic' is when the water solubility of an arbitrary part is lower than the other segment, the part is referred to as 'hydrophobic'. The hydrophobic part is desirably insoluble in water, and a soluble property is also acceptable as long as the water solubility is lower than the other parts.

In the second invention of the present invention, the amphiphilic compound is not particularly limited. The amphiphilic compound is preferably an amphiphilic compound having lipid, a surfactant, peptide, protein, and a saccharide segment in its structure. Moreover the amphiphilic compound is preferably an amphiphilic polymer.

Specific examples of the amphiphilic compound include peptides, proteins, sugars, and an analog thereof. For example, compounds obtained by providing the amphiphilicity to a targeting antibody, a basic peptide, or sugar are exemplified. In addition, the amphiphilic compound may be a hydrophilic polymer, and an analog thereof. The analog of the hydrophilic polymer may be exemplified by amphiphilic compounds for which a hydrophilic polymer is modified with a hydrophobic group, or surfactants, but may not be limited by those.

Examples of the hydrophilic polymer include polyamino acids and polysaccharides, such as polyethylene glycol, polyvinyl pyrrolidone, polyvinyl alcohol, polyethylenimine, polyacrylic acid, polymethacrylic acid, poly-1,3-dioxolan, 2-methacryloyloxyethyl phosphorylcholine polymer, poly-1,3,6-trioxane, polyasparagic acid, and the like. When the hydrophilic polymer is polyethylene glycol, Pluronic® which is commercially available from BASF is preferable as the amphiphilic polymer. Further, a block copolymer of polyethylene glycol and aliphatic polyester such as polylactic acid has biodegradability, thus particularly preferable.

As the surfactant, an anionic active agent such as alkyl sulfate salts e.g., sodium lauryl sulfate, ammonium lauryl sulfate, and sodium stearyl sulfate; and a nonionic active agent such as polyoxyethylene sorbitan mono-fatty acid ester, polyoxyethylene sorbitan di-fatty acid ester, polyoxyethylene glycerine mono-fatty acid ester, polyoxyethylene glycerine di-fatty acid ester, polyoxyethylene sorbit mono-fatty acid ester, polyoxyethylene sorbit di-fatty acid ester, and polyglycerine fatty acid ester, may be employed.

In the pharmaceutical preparation according to the second invention of the present invention, the biodegradable polymer is preferably any of aliphatic polyester, polyanhydride, polycarbonate, polyorthoester, poly-alpha-cyanoacrylic acid ester, polyphosphazene, polypeptide, and polyamino acid.

In the pharmaceutical preparation according to the second invention of the invention, the hydrophobic supermagnetic metal oxide is preferably supermagnetic metal oxide to which fatty acid or the salt thereof is bonded.

In order to easily form a complex and a particle with supermagnetic metal oxide by means of an amphiphilic substance and a matrix forming material used in the invention, the hydrophobic supermagnetic metal oxide of which the surface is coated with fatty acid such as oleic acid and the surface is hydrophobized, is particularly desirably employed.

In particular, a hydrophobic magnetite obtained by covering the above-mentioned magnetite Fe₃O₄, γ-Fe₂O₃ with fatty acid such as oleic acid and hydrophobizing the surface, is particularly preferable.

Such hydrophobic magnetite can be prepared in accordance with the well known method (for example, Biocatalysts, 5, 61 (1991)).

In the invention, fatty acid or fatty acid part of the fatty acid salt is preferably fatty acid having 12 to 22 carbon atoms. From the chemical stability standpoint, saturated fatty acid is more preferable, but unsaturated fatty acid may also be used. Examples of such fatty acid include saturated fatty acids such as lauric acid, myristic acid, stearic acid, palmitic acid, and behenic acid; and unsaturated fatty acids such as oleic acid. As a metal ion contained in the fatty acid salt, Na⁺ and Ca²⁺ can be exemplified. The iron salts may also be employed within the scope of not affecting the effect of the invention. From the solubility and easy availability points of view, a sodium salt is preferable.

The added amount of fatty acid is preferably more than the amount required for forming a monomolecular layer on the surface of a magnetite particle.

Such complex particle comprising the amphiphilic substance, the matrix forming material, and the hydrophobic supermagnetic metal oxide is highly useful as a therapeutic drug for thermotherapy (hyperthermia) for cancer and the like, in addition to the above-mentioned MRI contrast agent.

The administration mode and the applicable field of the pharmaceutical preparation according to the first and the second invention of the present invention are not particularly limited, but the invention may be applied variously. For example, for the pharmaceutical preparation, the drug may be used as an oral administration, parenteral administration, enteral administration, pulmonary administration, local administration (nose, skin, and eye), and administration via body cavities.

For the parenteral administration mode, the following administration modes may be particularly exemplified.
(1) an intravenous administration [the particle which releases an active substance such as a peptide drug, a cell-growth inhibitor, an immune stimulant, a growth factor e.g., colony-stimulating factor (leucocyte modulator) and a growth factor, under control, is circulated in blood for targeting liver, spleen, or marrow.],
(2) an intramuscular administration (e.g., administration of a depot preparation which provides an active substance such as peptide drug and hormone over a long period),
(3) an intraarticular administration (e.g., administration of an antirheumatic drug or an immunosuppressive drug during the therapy for arthritis),
(4) an intracavity administration (e.g., administration of a cell-growth inhibitor or a peptide drug for the therapy for cancer in peritoneum and pleural cavity), and
(5) a subcutaneous administration (e.g., administration of depot preparation of a cell-growth inhibitor for the therapy for skin cancer).

For the enteral administration, the following embodiments must be particularly considered.
(1) an administration of vitamin which is soluble in lipid,
(2) an absorption of lymph (e.g., targeting the lymph node by an active substance such as a cell-growth inhibitor, etc.),
(3) an administration of an antigen (e.g., oral immunization by using peyer's patch), and
(4) an administration of a peptide drug by using an M cell.

For the pulmonary administration, the following embodiments must be particularly considered.
(1) an aerosol or a blended aerosol (an aerosolized administration of an aqueous dispersion of a particulate preparation), and
(2) a drip infusion of the dispersion.

For the local administration, for example, the following embodiments are considered.
(1) an administration of dermatological drugs such as corticoid and an antifungal agent,
(2) an eye drop or an ophthalmological gel, for example, beta blocker, and
(3) a cosmetic similar to a liposome preparation.

Hereinafter, Examples will be shown, but the present invention is not limited by those Examples.

### (Reference Examples)

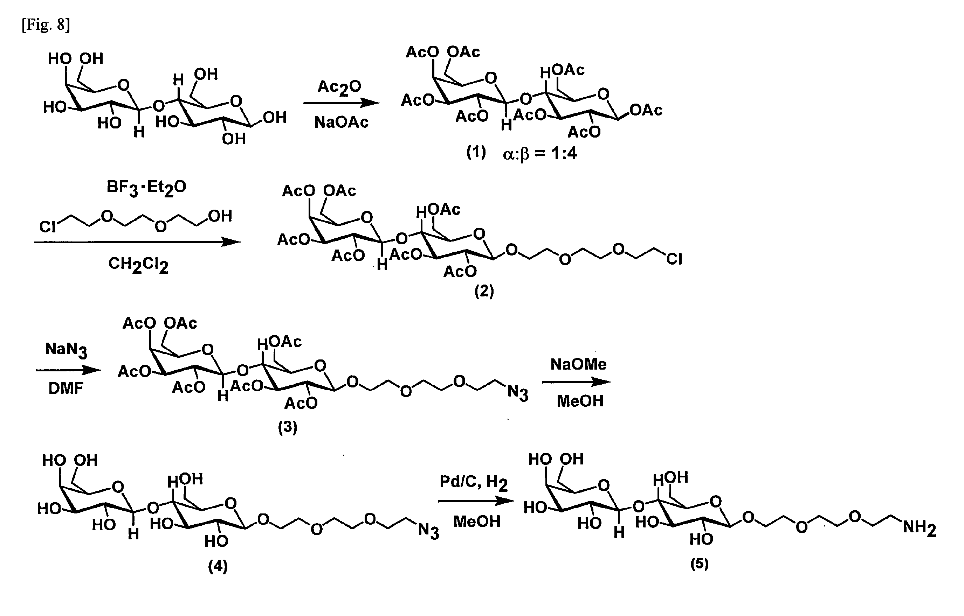

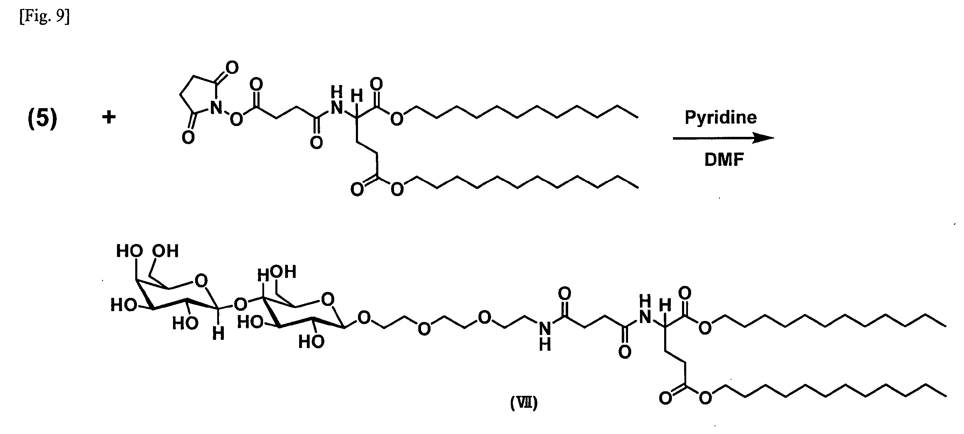

### [Reference Example 1] Synthesis of Amphiphilic Compound (VII) (synthesis scheme is shown in Figs. 8 and 9)

### 1-1. Synthesis of Compound (1)

Lactose monohydrate (5.0 g, 14.6 mmol), sodium acetate (1.3 g, 16.1 mmol), and acetic anhydride (20 mL) were mixed, and the mixture was refluxed under heating for 30 minutes. After the reaction, the acetic anhydride was distilled off under a reduced pressure, then methylene chloride (200 mL) was added, and after being washed with cold water and saturated brine, the organic layer was concentrated to obtain Compound (1) (9.4 g, 95% yield).

### 1-2. Synthesis of Compound (2)

The Compound (1) (4.0 g, 5.9 mmol) was suspended in a methylene chloride solution (50 mL) with molecular sieves sized 4Å at 0°C, and 2-[2-(2-chloroethoxy)ethoxy]-ethanol (9.9 mL, 67.9 mmol) was added thereto. The mixture solution was stirred at room temperature for 1 hour, then a trifluoroborane diethylether complex (8.4 ml, 66.0 mmol) was added dropwise to the mixture solution at 0°C, and then stirred for 18 hours at room temperature. After the reaction, methylene chloride (200 mL) was added, and the organic layer was washed with a 5% saturated aqueous solution of sodium bicarbonate. The organic layer was separated and dried over sodium sulfate, the organic solvent was removed under reduced pressure, and the residue was purified by column chromatography (n-hexane/ethyl acetate = 1/1) to obtain Compound (2) (2.6 g, 56% yield).

### 1-3. Synthesis of Compound (3)

Sodium azide (3 g, 4.6 mmol) was added to an N,N-dimethylformamide solution (50 mL) of Compound (2) (2.6 g, 3.3 mmol), and stirred at 65°C for 5 days. The solvent was distilled off under reduced pressure, the residue was dissolved in ethyl acetate (100 mL), and after separating out the insolubles by filtration, the resultant was concentrated under reduced pressure to obtain Compound (3) (2.5 g, 95% yield).

### 1-4. Synthesis of Compound (4)

The Compound (3) (2.5 g, 3.09 mmol) was dissolved in methanol (40 mL), and sodium methoxide of catalyst amount was added thereto. After the reaction (5 h, room temperature), the system was neutralized with amberlite IRC50 (5 g), separated by filtration, and then concentrated under reduced pressure to obtain Compound (4) (1.35 g, 85% yield).

### 1-5. Synthesis of Compound (5)

To a methanol solution (10 mL) of the Compound (4) (2.5 g, 3.09 mmol), 10% Pd/C (30 mg) was added, and the mixture was stirred in a hydrogen atmosphere of normal pressure for 1 hour. The reaction solution was separated by filtration, and then concentrated to obtain Compound (5) (1.35 g, 85% yield) as a syrup. 1-6. Synthesis of Amphiphilic Compound (VII)

The Compound (5) (1.3 g, 2.52 mmol) was dissolved in a mixture solvent (6 mL:0.7 mL) of N,N-dimethylformamide and pyridine. Thereto, glutamate dodecyl ester-type active succinate ester (1.3 g, 1.9 mmol) was added, and stirred at 40°C for 6 hours. After the reaction, the solvent was distilled off under reduced pressure, and the residue was directly purified by silica gel column chromatography (chloroform/methanol = 11/1) to obtain the amphiphilic compound (VII) (100 mg, 0.4% yield).

¹H-NMR (400 MHz,CDCl₃), δ 7.41 - 7.30 (m, 2H), δ 4.45 (d, 1H, J = 7.6 Hz), δ 4.36 - 4.30 (m, 1H, H-1), δ 4.06 - 3.31 (m, 29H), δ 2.68 - 2.52 (m, 4H), δ 2.41 - 2.30 (m, 2H), δ 2.20 - 2.08 (m, 1H), δ 2.05 - 1.92 (m, 1H), δ 1.71 - 0.80 (m, 46H). MALDI-TOF for C₅₁H₉₄N₂O₁₉ (MW = 1062.29): m/z = 1062.22 [M+Na]⁺. Elemental Anal.Calcd for C₅₁H₉₄N₂O₁₉.H₂O: C, 59.61, H, 10.09, N, 2.44%; Found: C, 59.81, H, 10.17, N, 2.31%.

### [Reference Example 2]

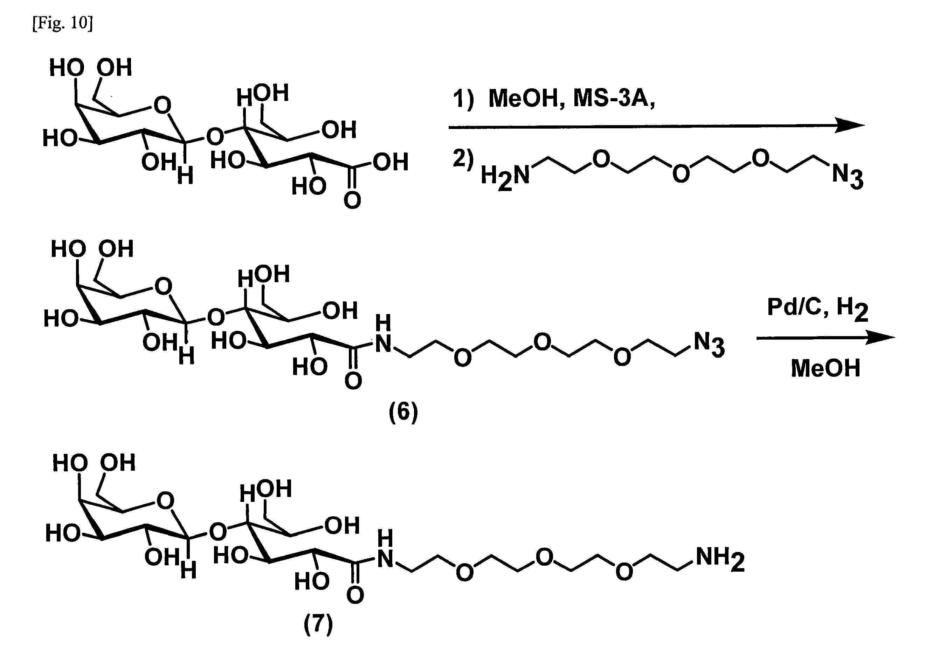

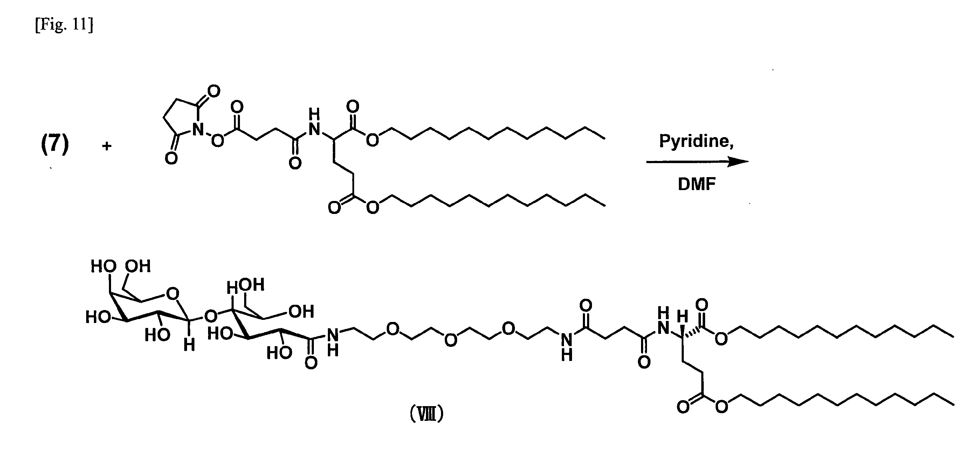

### Synthesis of Amphiphilic Compound (VIII): synthesis scheme of the amphiphilic compound (VIII) is shown in Figs. 10 and 11.

### 2-1. Synthesis of Compound (6)

Lactobionic acid (7.0 g, 19.5 mmol) was dissolved in methanol (70 mL) under an argon stream at room temperature, molecular sieves sized 3Å were charged thereto, and refluxed overnight under heating in an azeotropic condition. Subsequently, 11-azido-3.6.9-trioxaundecan-1-amine (4.26 mL, 21.5 mmol) was added dropwise at room temperature, stirred overnight, and the solvent of thus-obtained reaction solution was distilled off under reduced pressure. The obtained residue was dissolved in methanol (5 mL), and then chloroform (30 mL) and tetrahydrofuran (100 mL) were added in the mentioned order to obtain white precipitates. After washing the precipitates with tetrahydrofuran, the product was dissolved in water (20 mL), subjected to freeze-drying, and Compound (6) (5.56 g, 51% yield) was obtained as a white solid.

### 2-2. Synthesis of Compound (7)

The Compound (6) (2.0 g, 3.6 mmol) was dissolved in methanol (40 mL) at room temperature, 10% -Pd/C (0.18 mmol) was added thereto, and subjected to hydrogen substitution at normal pressure. After the overnight stirring, the catalyst was removed by celite filtration, and the solvent was distilled off under reduced pressure. The obtained residue was dissolved in water (20 mL) and subjected to freeze-drying to obtain Compound (7) (1.86 g, 97% yield) as white individuals.

### 2-3. Synthesis of Amphiphilic Compound (VIII)

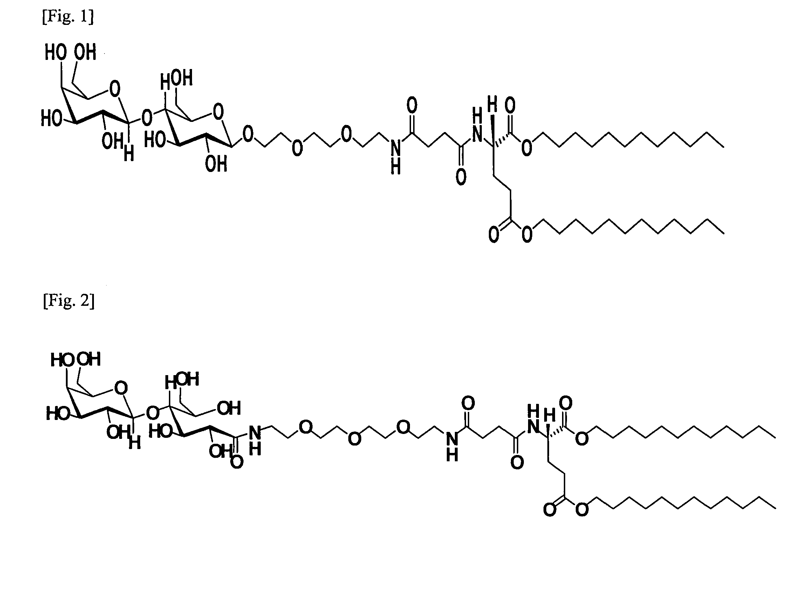

The Compound (7) (0.11 g, 0.2 mmol) was dissolved in N,N-dimethylformamide/pyridine (3 mL/4 mL) under an argon stream at room temperature, glutamate dodecyl ester-type active succinate ester (0.14 g, 0.2 mmol) was added thereto, and the mixture was stirred overnight. The solvent of the obtained reaction solution was distilled off under reduced pressure. The obtained residue was purified by silica gel column chromatography (chloroform/methanol = 3/1) to obtain the amphiphilic compound (VIII) (0.1 g, 43.8% yield) having the structure below. In Fig. 2, the structure of the amphiphilic compound (VIII) is shown.

¹H-NMR (400 MHz,DMSO-d⁶) δ 4.80 - 4.30 (2H, m), δ 4.67 (1H, d, J = 7.3 Hz), δ 4,44 (1H, dd, J = 5.4 and 8.8 Hz), δ 4.35 (1H,d, J = 2.4 Hz), δ 4.22 (1H, dd, J = 3.9 and 1.5 Hz), δ 4.16 - 4.04 (4H, m), 3.94 - 3.84 (2H, m), δ 3.84 - 3.75 (4H, m), δ 3.64 (8H, m), δ 3.60 - 3.53 (5H, m), δ 3.50 - 3.34 (5H, m), δ 2.57 - 2.47 (4H, m), δ 2.42 (2H, t, J = 7.3 Hz), δ 2.19 - 1.91 (2H, m), δ 1.63 (4H, m), δ 1.30 (36H, m), δ 0.90 (6H, t, J = 6.8 Hz). MALDI-TOF for C₅₃H₉₉N₃O₂0 (MW= 1098.36): m/z = 1120.634 [M+Na]⁺.

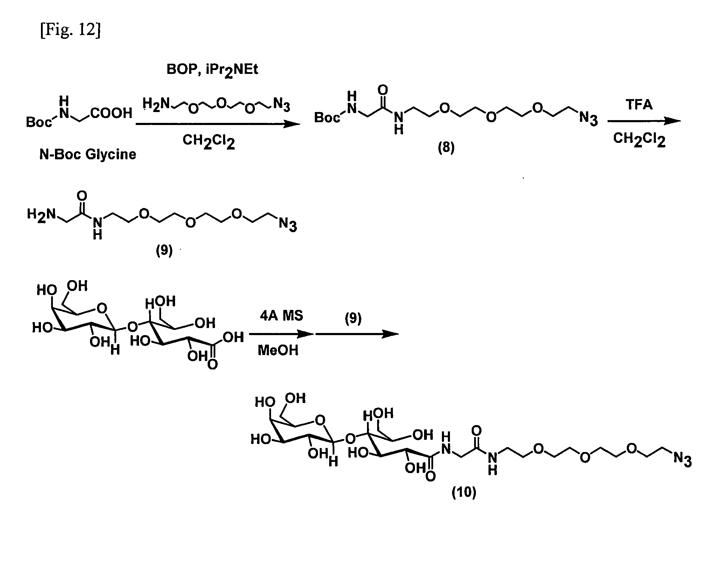

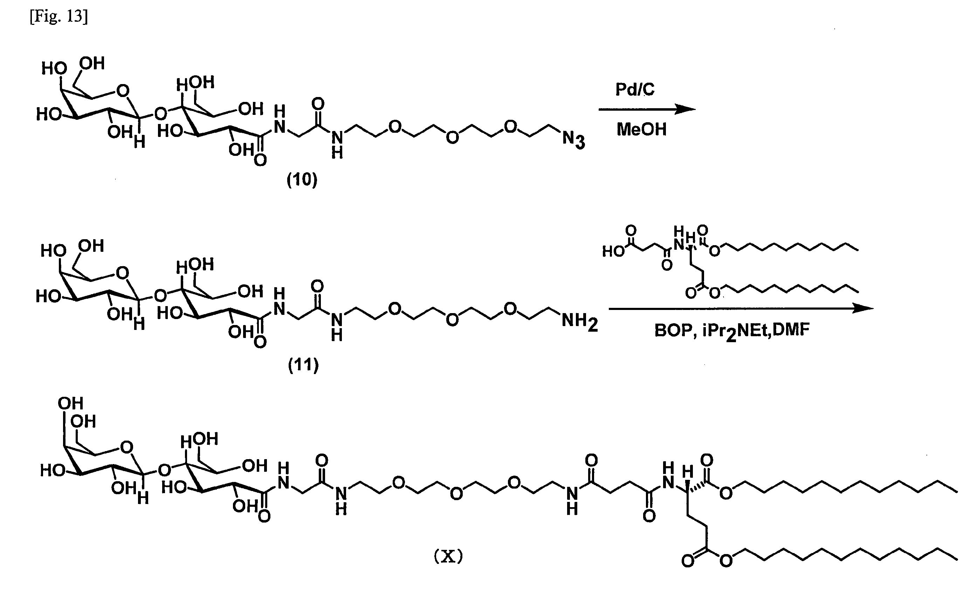

### [Reference Example 3] Synthesis of Amphiphilic Compound (X) (synthesis scheme is shown in Figs. 12 and 13)

### 3-1. Synthesis of Compound (8)

N-Boc-Glycine (1.4 g, 8.0 mmol), 11-Azido-3.6.9-trioxaundecan-1-amine (1.92 g, 8.8 mmol) and 1-Ethyl-3-(3-dimethylaminopropyl)-carbodiimide Hydrochloride (2.3 g, 12.0 mmol) were dissolved in methylene chloride (16 ml) under an argon stream at room temperature, and the mixture was stirred at room temperature for 3 hours. The reaction solution was added with water and extracted with ethyl acetate. The organic layer was dried over anhydrous sodium sulfate, and then the solvent was distilled off under reduced pressure. The obtained residue was purified by silica gel column chromatography (chloroform/methanol = 10/1) to obtain Compound (8) (2.1 g, 70% yield) having the structure below.

### 3-2. Synthesis of Compound (9)

The Compound (8) (2.1 g, 5.6 mmol) was dissolved in methylene chloride (11.2 mL) at room temperature. Thereto, trifluoroacetic acid (3.2 g, 28.0 mmol) was added and stirred overnight. The solvent of the reaction solution was distilled off under reduced pressure, and then the obtained residue was added with an IN aqueous solution of sodium hydroxide. After confirming that the pH is 11 or above, extraction with chloroform was carried out. The organic layer was dried over anhydrous sodium sulfate, and the solvent was distilled off under reduced pressure. The obtained residue was purified by amine silica gel column chromatography (chloroform/methanol = 10/1) to obtain Compound (9) (1.28 g, 83% yield) having the structure below.

### 3-3. Synthesis of Compound (10)

Lactobionic acid (1.51 g, 4.2 mmol) was dissolved in methanol (21 mL) under an argon stream at room temperature, molecular sieves 3Å were charged thereto, and refluxed overnight under heating in an azeotropic condition. Subsequently, a methanol solution (5.0 mL) of the Compound (9) (1.28 g, 4.65 mmol) was added dropwise to the reaction solution at room temperature, and stirred overnight at room temperature for 3 hours. After distilling off the solvent of the reaction solution under reduced pressure, the obtained residue was purified by silica gel column chromatography (chloroform/methanol = 1/1), and Compound (10) having the structure below was obtained (1.85 g, 71% yield).

### 3-4. Synthesis of Compound (11)

To a methanol solution (30 mL) of the Compound (10) (1.85 g, 3.01 mmol), 10% Pd/C (100 mg) was added, and the mixture was stirred in a hydrogen atmosphere of normal pressure for 3 hours. The reaction solution was filtered through celite, and then concentrated to obtain white amorphous Compound (11) (1.74 g, 98% yield).

### 3-5. Synthesis of Amphiphilic Compound (X)

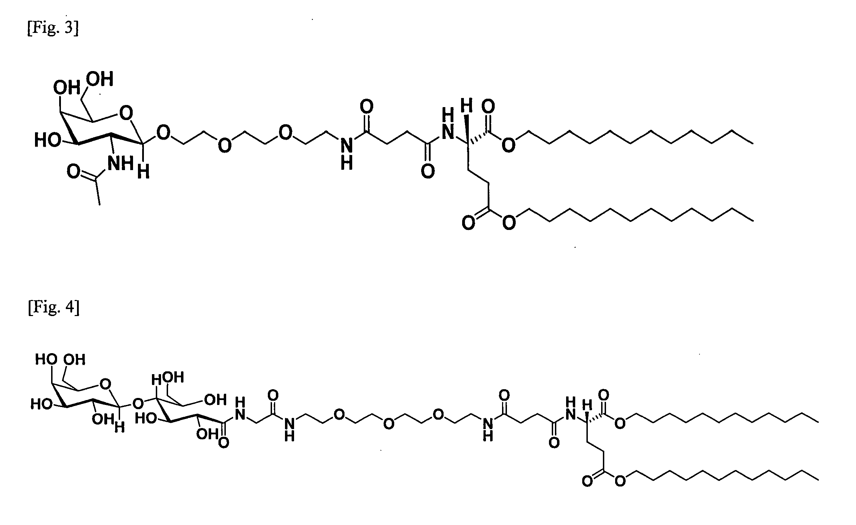

The Compound (11) (0.4 g, 0.68 mmol) and glutamate dodecyl ester-type succinic acid (0.44 g, 0.75 mmol) were dissolved in N,N-dimethylformamide (6.8 mL), then N,N-diisopropylethylamine (0.26 g, 2.04 mmol) and a BOP reagent (0.45 g, 1.02 mmol) were added thereto, and the mixture was stirred at room temperature for 4 hours. After the reaction, the solvent was distilled off under reduced pressure, and the residue was directly purified by silica gel column chromatography (chloroform/methanol = 4/1) to obtain the amphiphilic compound (X) (87 mg, 11 % yield). In Fig. 4, the structure of the amphiphilic compound (X) is shown.

¹H-NMR (400 MHz, CD₃OD), δ 4.49 (d, 1H, J = 7.6Hz), δ 4.45 - 4.40 (m, 2H), δ 4.24 - 4.19 (m, 1H), δ 4.16 - 4.04 (m, 4H), δ 3.99 - 3.93 (m, 2H), δ 3.91 - 3.85 (m, 2H), δ 3.82 - 3.75 (m, 4H), δ 3.72 - 3.47 (m, 16H), δ 3.42 - 3.33 (m, 4H), δ 2.57 - 2.46 (m, 4H), δ 2.43 (t, 2H, J = 7.2 Hz), δ 2.20 - 2.10 (m, 1H), δ 2.00 - 1.90 (m, 1H), δ 1.71 - 1.59 (m, 4H), δ 1.42 - 1.23 (m, 36H), δ 0.90 (t, 6H, J = 6.8 Hz).

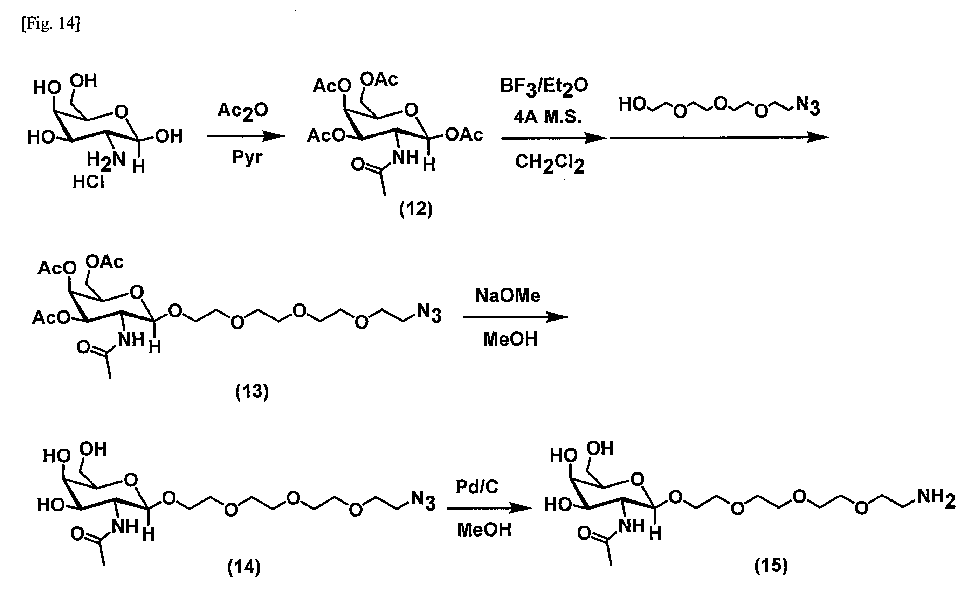

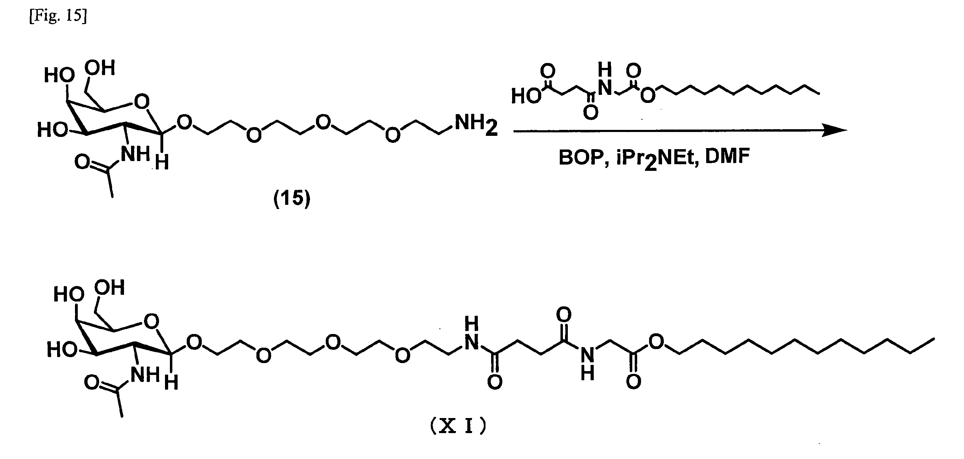

### [Reference Example 4] Synthesis of Amphiphilic Compound (XI) (synthesis scheme is shown in Figs. 14 and 15)

### 4-1. Synthesis of Compound (12)

To D-galactosamine hydrochloride salt (5.0 g, 23.2 mmol), pyridine (48 mL) was added and suspended, the suspension was cooled to 0°C, and acetic anhydride (60 mL) was added dropwise thereto. After the 48 hours stirring at room temperature, the reaction solution was cooled to 0°C and poured into distilled water (700 mL). After stirring vigorously at 4°C, thus obtained white precipitate was separated by filtration. The obtained white solid was dried under reduced pressure to obtain Compound (12) (7.2 g, 80% yield) having the structure below.

### 4-2. Synthesis of Compound (13)

The Compound (12) (3.9 g, 10.0 mmol) was dissolved in methylene chloride (60 mL), then molecular sieves 4Å were added, stirred for 1 hour, and thoroughly dried. Thereafter, the reaction solution was cooled to 0°C, and boron trifluoride diethylether complex (4.3 g, 30.0 mmol) was added dropwise thereto. After the overnight stirring at room temperature, the reaction solution was added with 1-Azido-3.6.9-trioxaundecan-11-ol (4.38 g, 20.0 mmol), and further stirred at room temperature for 48 hours. The reaction solution was cooled to 4°C and poured into 5% aqueous sodium carbonate solution (200 mL). After separating the molecular sieve 4Å by filtration, chloroform was added to isolate the organic layer. The organic layer was washed with brine, then dried over anhydrous sodium sulfate, and the solvent was distilled off under reduced pressure. The obtained residue was purified by silica gel column chromatography (ethyl acetate/methanol = 10/1) to obtain Compound (13) (3.7 g, 66% yield) having the structure below.

### 4-3. Synthesis of Compound (14)

The Compound (13) (3.62 g, 6.6 mmol) was dissolved in methanol (33 mL), and a methanol solution (0.3 mL) of sodium methoxide was added dropwise thereto. After the 4 hours stirring at room temperature, the mixture was added with a weak acid ion exchange resin (Amberlite IRC50 (5.0g)) to be neutralized and separated by filtration. The residue obtained by concentration under reduced pressure was purified by silica gel column chromatography (chloroform/methanol = 2/1), and Compound (14) having the structure below was obtained (2.3 g, 80% yield).

### 4-4. Synthesis of Compound (15)

To a methanol solution (27 mL) of the Compound (14) (2.3 g, 5.3 mmol), 10% Pd/C (100mg) was added, and the mixture was stirred in a hydrogen atmosphere of normal pressure for 3 hours. The reaction solution was filtered through celite, and then concentrated under reduced pressure to obtain Compound (15) (2.1 g, quant) having the structure below as a syrup.

### 4-5. Synthesis of Amphiphilic Compound (XI)

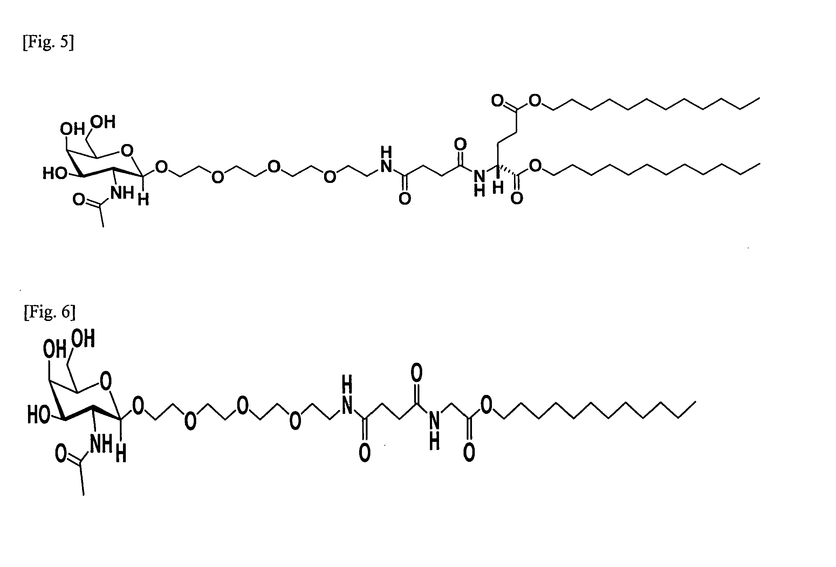

The Compound (15) (0.32 g, 0.8 mmol) and glycine dodecyl ester-type succinic acid (0.30 g, 0.88 mmol) were dissolved in N,N-dimethylformamide (8.0 mL), then N,N-diisopropylethylamine (0.31 g, 2.4 mmol) and a BOP reagent (0.53 g, 1.2 mmol) were added thereto, and the mixture was stirred at room temperature for 4 hours. After the reaction, the solvent was distilled off under reduced pressure, and the residue was directly purified by silica gel column chromatography (chloroform/methanol = 4/1) to obtain the amphiphilic compound (XI) (280 mg, 48% yield). In Fig. 5, the structure of the amphiphilic compound (XI) is shown.

¹H-NMR (400 MHz, CD₃OD), δ 4.45 (d, 1H, J = 7.6 Hz), δ 4.13 (t, 2H, J = 6.6 Hz), δ 3.98 - 3.89 (m, 4H), δ 3.84 - 3.82 (m, 1H), δ 3.78 - 3.68 (m, 3H), δ 3.67 - 3.46 (m, 14H), δ 3.40 - 3.34 (m, 2H), δ 2.59 - 2.48 (m, 4H), δ 2.00 (s, 3H), 8 1.70 - 1.60 (m, 2H), δ 1.40 - 1.23 (m, 18H), δ 0.91 (t, 3H, J = 6.8Hz).

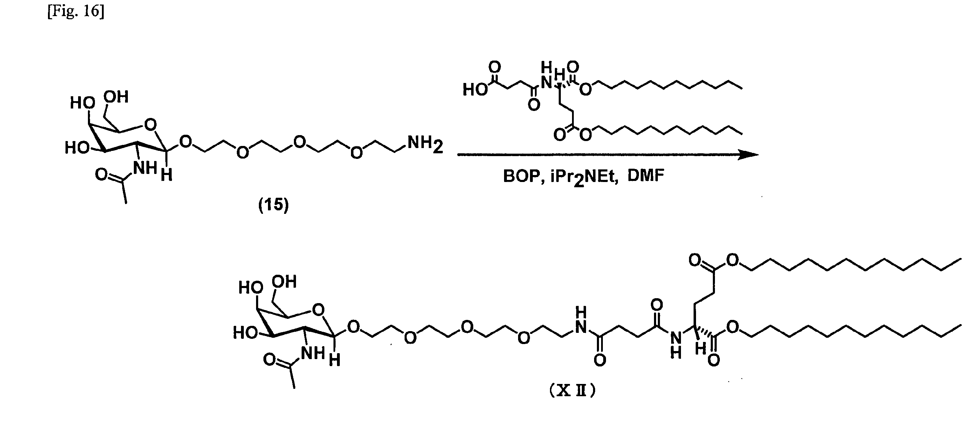

### [Reference Example 5] Synthesis of Amphiphilic Compound (XII) (synthesis scheme is shown in Fig. 16)

### 5-1. Synthesis of Amphiphilic Compound (XII)

The Compound (15) (0.20 g, 0.5 mmol) and glutamate dodecyl ester-type succinic acid (0.32 g, 0.55 mmol) were dissolved in N,N-dimethylformamide (5.0 mL), then N,N-diisopropylethylamine (0.19 g, 1.5 mmol) and a BOP reagent (0.33 g, 0.75 mmol) were added thereto, and the mixture was stirred at room temperature for 4 hours. After the reaction, the solvent was distilled off under reduced pressure, and the residue was directly purified by silica gel column chromatography (chloroform/methanol = 3/1) to obtain the amphiphilic compound (XII) (135 mg, 28% yield). In Fig. 6, the structure of the amphiphilic compound (XII) is shown.

¹H-NMR (400 MHz, CD₃OD), δ 4.47 - 4.40 (m, 2H), δ 4.19 - 4.04 (m, 4H), δ 3.98 - 3.91 (m, 2H), δ 3.86 - 3.47 (m, 18H), δ 3.37 (t, 2H, J = 7.6 Hz), δ 2.61 - 2.39 (m, 6H), δ 2.20 - 2.09 (m, 1H), δ 2.03 - 1.90 (m, 1H), δ 2.00 (s, 3H), δ 1.70 - 1.58 (m, 4H), δ 1.44 - 1.23 (m, 36H), δ 0.91 (t, 6H, J = 6.8 Hz).

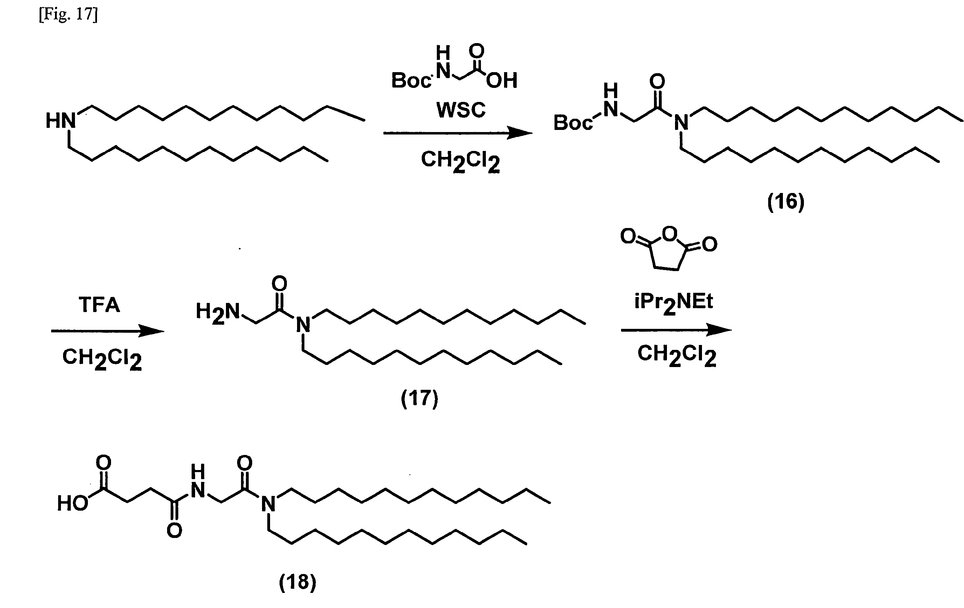

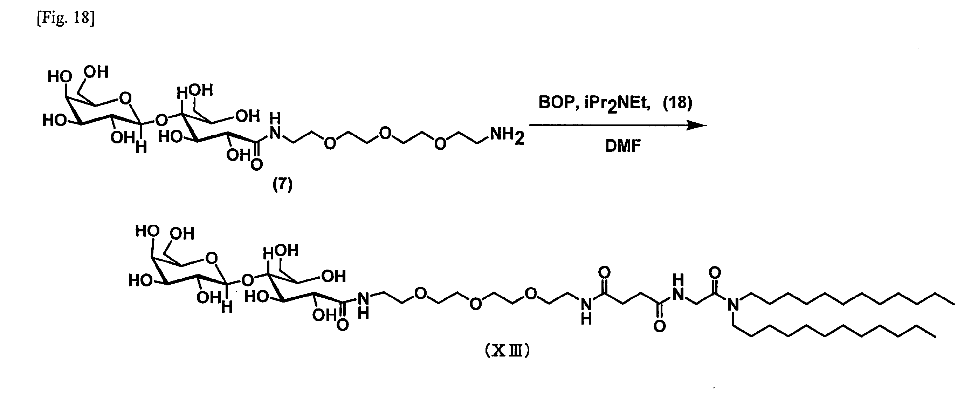

### [Reference Example 6] Synthesis of Amphiphilic Compound (XIII) (synthesis scheme is shown in Figs. 17 and 18)

### 6-1. Synthesis of Compound (16)

Didodecylamine (1.76 g, 5.0 mmol), N-Boc Glycine (1.1 g, 6.0 mmol), and 1-Ethyl-3-(3-dimethylaminopropyl)-carbodiimide Hydrochloride (1.44 g, 7.5 mmol) were dissolved in methylene chloride (25 ml) under an argon stream at room temperature, and the mixture was stirred at room temperature for 5 hours. The reaction solution was added with water and extracted with ethyl acetate. Thereafter, the organic layer was dried over anhydrous sodium sulfate, and the solvent was distilled off under reduced pressure. The obtained residue was purified by silica gel column chromatography (ethyl acetate/n-hexane = 1/1) to obtain Compound (16) (2.55 g, quant) having the structure below.

### 6-2. Synthesis of Compound (17)

The Compound (16) (2.55 g, 5.0 mmol) was dissolved in methylene chloride (10 mL) at room temperature, then trifluoroacetic acid (2.85 g, 25.0 mmol) was added thereto and stirred for 7 hours. The solvent of the reaction solution was distilled off under reduced pressure, and then the obtained residue was added with an 1N aqueous solution of sodium hydroxide. After confirming that the pH is 11 or above, extraction with chloroform was carried out. The organic layer was dried over anhydrous sodium sulfate, and the solvent was distilled off under reduced pressure. The obtained residue was purified by amine silica gel column chromatography (chloroform/methanol = 10/1) to obtain Compound (17) (1.46 g, 71% yield) having the structure below.

### 6-3. Synthesis of Compound (18)

The Compound (17) (1.4 g, 3.4 mmol) and succinic anhydride (0.68 g, 6.8 mmol) were dissolved in methylene chloride (6.8 mL) under an argon stream, then N,N-diisopropylethylamine (1.76 g, 13.6 mmol) was added, and the mixture was stirred at room temperature for 4 hours. The solvent of the reaction solution was distilled off under reduced pressure, and then the obtained residue was added with an IN aqueous hydrochloric acid solution. After confirming that the pH is 3 or less, extraction with ethyl acetate was carried out. The obtained organic layer was washed with brine, then dried over anhydrous sodium sulfate, and the solvent was distilled off under reduced pressure. The obtained residue (white solid) was purified by silica gel column chromatography (ethyl acetate/methanol = 10/1) to obtain Compound (18) (1.74 g, quant) having the structure below as a white solid.

### 6-4. Synthesis of Amphiphilic Compound (XIII)

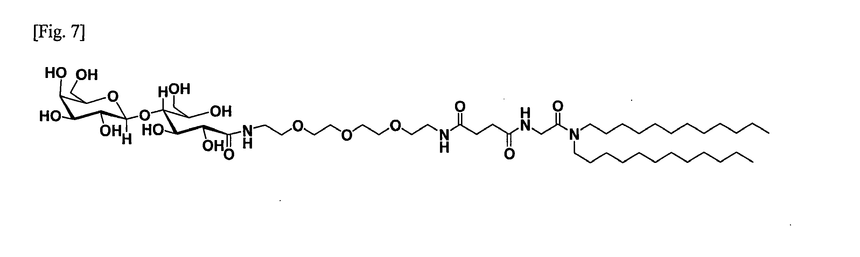

The Compound (7) (0.40 g, 1.0 mmol) and the Compound (18) (0.56 g, 1.1 mmol) were dissolved in N,N-dimethylformamide (10.0 mL), then N,N-diisopropylethylamine (0.39 g, 3.0 mmol) and a BOP reagent (0.66 g, 1.5 mmol) were added thereto, and the mixture was stirred at room temperature for 6 hours. After the reaction, the solvent was distilled off under reduced pressure, and the residue was directly purified by silica gel column chromatography (chloroform/methanol = 4/1) to obtain the amphiphilic compound (XIII) (112 mg, 12% yield). In Fig. 7, the structure of the amphiphilic compound (XIII) is shown.

¹H-NMR (400 MHz, CD₃OD), δ 4.49 (d, 1H, J = 7.6 Hz), δ 4.36 (d, 1H, J = 2.8 Hz), δ 4.23 (dd, 1H, J = 3.6 and 2.8 Hz), δ 4.05 (s, 2H), δ 3.95 - 3.91 (m, 1H), δ 3.90 - 3.85 (m, 1H), δ 3.84 - 3.75 (m, 4H), δ 3.73 - 3.40 (m, 19H), δ 3.40 - 3.25 (m, 5H), δ 2.62 - 2.48 (m, 4H), δ 1.68 - 1.50 (m, 4H), δ 1.42 - 1.21 (m, 38H), δ 0.91 (t, 6H, J = 6.8 Hz).

### [Reference Example 7] Particle Size Measuring Method

The size of the prepared particle was measured using a dynamic light scattering (DSL) method. The measurement was carried out using ZE-TASIZER 3000HSA manufactured by MELVERN Instruments, and the analysis was done with the application of Multi Exponential Analysis to determine the average particle size of a volume distribution.

### [Reference Example 8] A Method of Confirming Glycosylation on Particle Surface

The prepared particulate preparation solution was diluted 5-fold, and filtered through a filter of 0.45 µm and 0.20 µm. Thereafter, the resultant was settled by centrifugation at 13,000 rpm for 1 h to remove the water soluble component, and PBS was added to pellets obtained by removing the supernatant for re-dispersion. To the solution, lectin (5 mg/mL, 5 µL) was added, the mixture was incubated at 37°C for 1 hour, and then the particle size was measured. The particle size of the ones not added with lectin was measured in the same manner. The size of each particle was compared to evaluate the aggregation, and the particles showing aggregation were judged as that the saccharides are presented on the surface.

### [Reference Example 9] Evaluation of Protein Adsorbability on Particle Surface

The prepared particulate preparation solution was diluted 5-fold, and filtered through a filter of 0.45 µm and 0.20 µm. Thereafter, the resultant was settled by centrifugation at 13,000 rpm for 1 h to remove the water soluble component, and PBS was added to pellets obtained by removing the supernatant for re-dispersion. To the solution, BSA/PBS (0.1 mg/mL, 0.5 mL) was added, the mixture was incubated at 37°C for 1 hour, and then the particle size was measured. The particle size of the ones not including BSA was measured in the same manner. The size of each particle was compared to evaluate the aggregation. When the aggregation was observed, it was judged as that the protein is adsorbed on the surface, and when the aggregation was not observed, it was judged as that the protein is not adsorbed.

### [Reference Example 10] Fluorescent Magnetite Preparation Method

Oleylic magnetite (31.2 mg Fe) and aminopropyltriethoxysilane (33 mg) were added to toluene (25 mL), and refluxed for 1 hour at an oil bath temperature of 130°C. Thus obtained solution was slowly added to 100 mL of ethanol on a magnet for precipitation, the supernatant was removed, and the precipitate was re-dissolved in tetrahydrofuran. The obtained tetrahydrofuran solution (25 mL) of aminated magnetite was added with FITC (15 mg), and the mixture was stirred at room temperature for 30 minutes. Thus obtained solution was slowly added to 100 mL of ethanol on a magnet for re-precipitation, the supernatant was removed, and the precipitate was re-dissolved in tetrahydrofuran. This manipulation was repeated twice. The amount of iron in the solution obtained was quantified to determine the yield.

### [Reference Example 11] Evaluation of fluorescent magnetite-enclosed particulate Preparation Uptake by Hepatic cells when administered to Mouse

Prepared fluorescent magnetite-enclosed particulate preparation was dispersed in a physiological saline, and the solution was injected via tail vein of an ICR mouse. 30 minutes after the injection, hepatic parenchymal cells were isolated from the ICR mouse by a collagenase perfusion technique. The obtained mouse hepatic parenchymal cell 5x105 pieces were suspended in 1 mL of PBS (Phosphate Buffer Saline). The sample was analyzed using a Flow cytometry (apparatus: FACS Calibur manufactured by BECTON DICKINSON). According to the analysis with Flow cytometry, a group administered with the fluorescent magnetite-enclosed particulate preparation and a group for control (non-administered) were compared to observe the shift at a peak position, thus the uptake of fluorescent magnetite-enclosed particulate preparation by hepatic parenchymal cells was evaluated.

### [Example 1] Preparation of Particulate Preparation comprising Amphiphilic Compound (VII)

The amphiphilic compound (VII) (0.125mg) was dissolved in 250 µL of acetone/methanol (10/2, v/v), this was added to 1 mL of PBS, and the organic solvent was removed using an evaporator to obtain a colloidal solution. As a result of measuring the particle size according to the method of Reference Example 7, the average particle size was 425 nm. In addition, the presence of saccharides on this particle surface and the protein non-adsorbability were confirmed in accordance with the methods of Reference Examples 8 and 9.

### [Example 2] Preparation of PLGA matrix particulate Preparation containing Amphiphilic Compound (VII) as constituent element

The amphiphilic compound (VII) (0.25 mg) and PLGA matrix (1.25 mg) were dissolved in 500 µL of acetone/methanol (10/2, v/v), this was added to 1 mL of PBS, and the organic solvent was removed using an evaporator to obtain a colloidal solution. As a result of measuring the particle size according to the method of Reference Example 7, the average particle size was 138 nm. In addition, the presence of saccharides on this particle surface and the protein non-adsorbability were confirmed in accordance with the methods of Reference Examples 8 and 9.

### [Example 3] Preparation of Fluorescence labeling HSPC liposome particulate Preparation containing Amphiphilic Compound (VII) as constituent element

Rhodamine PE (0.01 mg), the amphiphilic compound (VII) (0.5 mg), and HSPC (hydrogenated soybean lecithin) (5 mg) which is a material for forming liposome, were dissolved in chloroform (1 mL), argon was sprayed, and the solvent was distilled off to prepare a thin membrane. The membrane was dried for 3 hours at room temperature under reduced pressure, PBS solution (0.5 mL) was added thereto, and vigorously stirred for 5 minutes using a vortex mixer. The ultrasonic irradiation (20 W, 4 minutes, irradiated for 1 second, paused for 1 second) was carried out, and a fluorescent material not incorporated in liposome was taken using GPC (gel permeation chromatography). As a result of measuring the particle size according to the method of Reference Example 7, the average particle size was 84 nm.

### [Example 4] Preparation of Particulate Preparation comprising Amphiphilic Compound (VIII)

The amphiphilic compound (VIII) (1.0 mg) was dissolved in 250 µL of acetone/methanol (10/2, v/v), this was added to 1 mL of PBS, and the organic solvent was removed using an evaporator to obtain a colloidal solution. As a result of measuring the particle size according to the method of Reference Example 7, the average particle size was 119 nm. In addition, the presence of saccharides on this particle surface and the protein non-adsorbability were confirmed in accordance with the methods of Reference Examples 8 and 9.

### [Example 5] Preparation of PLGA matrix particulate Preparation containing Amphiphilic Compound (VIII) as constituent element

The amphiphilic compound (VIII) (1.5 mg) and PLGA (1.5 mg) were dissolved in 500 µL of acetone/methanol (9/1, v/v), this was added to 1 mL of PBS, and the organic solvent was removed using an evaporator to obtain a colloidal solution. As a result of measuring the particle size according to the method of Reference Example 7, the average particle size was 163 nm. In addition, the presence of saccharides on this particle surface and the protein non-adsorbability were confirmed in accordance with the methods of Reference Examples 8 and 9.

### [Example 6] Preparation of PLGA matrix particle enclosing magnetite and containing Amphiphilic Compound (VIII) as constituent element

Oleylic magnetite (0.1 mg Fe), PLGA matrix (0.15 mg), and the amphipatic compound (VIII) (0.25 mg) were mixed and dissolved in 250 µL of tetrahydrofuran/methanol (10/2, v/v). This was added to PBS (1 mL), and the organic solvent was removed using an evaporator. Thereafter, the resultant was filtered through a filter of 0.45 µm and 0.2 µm, settled by centrifugation at 13,000 rpm for 1 h to remove the water soluble component, and PBS was added to pellets obtained by removing the supernatant for re-dispersion. As a result of measuring the particle size according to the method of Reference Example 7, the average particle size was 93 nm. In addition, the presence of saccharides on this particle surface and the protein non-adsorbability were confirmed in accordance with the methods of Reference Examples 8 and 9.

### [Example 7] (Preparation of Magnetite Particle with Surface-modified Amphiphilic Compound (VIII))

Oleylic magnetite (0.2 mg Fe) and the amphipatic compound (VIII) (0.2 mg) were mixed and dissolved in 200 µL of tetrahydrofuran/methanol (10/2, v/v). This was added to PBS (1 mL), and the organic solvent was removed using an evaporator. Thereafter, the resultant was filtered through a filter of 0.45 µm and 0.2 µm, settled by centrifugation at 13,000 rpm for 1 h to remove the water soluble component, and PBS was added to pellets obtained by removing the supernatant for re-dispersion. As a result of measuring the particle size according to the method of Reference Example 7, the average particle size was 166 nm. In addition, the presence of saccharides on this particle surface and the protein non-adsorbability were confirmed in accordance with the methods of Reference Examples 8 and 9.

Hereinafter, particulate preparations were prepared in the same manner with the use of amphiphilic compounds (X), (XI), (XII), and (XIII).

**[Table 1]**

| Ex. | Amphiphilic Compound | PLGA | Enclosed Substance* | Solvent | Non-Solvent | Particle Size |
|---|---|---|---|---|---|---|
| 8 | (X) 1.0 mg | 1.25 mg | - | Acetone/ Methanol (10/2,v/v) 0.5 mL | PBS (1mL) | 99 nm |
| 9 | (X) 0.25mg | 0.15 mg | OM 0.1 mgFe | THF/ Methanol (10/2,v/v) 0.25 mL | PBS (1mL) | 148 nm |
| 10 | (X) 1.0 mg | 0.5 mg | FOM 0.1 mgFe | THF/ Methanol (10/2,v/v) 0.3 mL | PBS (1mL) Filtered Centrifuged | 176 nm |
| 11 | (XI) 0.25 mg | 0.25 mg | - | Acetone/ Methanol (10/2,v/v) 0.2 mL | PBS (1mL) | 126 nm |
| 12 | (XI) 0.25 mg | 0.15 mg | OM 0.1 mgFe | THF/ Methanol (10/2,v/v) 0.125 mL | PBS (1mL) | 197 nm |
| 13 | (XI) 0.1 mg | 0.025 mg | FOM 0.025 mgFe | THF/ Methanol (10/2,v/v) 0.2 mL | PBS (1mL) Filtered Centrifuged | 192 nm |
| 14 | (XII) 1.0 mg | 1 mg | - | Acetone/ Methanol (10/2,v/v) 0.125 mL | PBS (1mL) | 165 nm |
| 15 | (XII) 0.25 mg | 0.15 mg | OM 0.1 mgFe | THF/ Methanol (10/2,v/v) 0.25 mL | PBS (1mL) | 74 nm |
| 16 | (XII) 1.0 mg | 0.5 mg | FOM O.lmgFe | THF/ Methanol (10/2,v/v) 0.3 smL | PBS (1mL) | 173nm |
| 17 | (XIII) 1.0 mg | 1 mg | - | Acetone/ Methanol (10/2,v/v) 0.25 mL | PBS (1mL) | 112 nm |
| 18 | (XIII) 0.25 mg | 0.15 mg | OM 0.1 mgFe | THF/ Methanol (10/2,v/v) 0.25 mL | PBS (1mL) | 110 nm |
| 19 | (XIII) 1.0 mg | 0.5 mg | FOM O.lmgFe | THF/ Methanol (10/2,v/v) 0.3 mL | PBS (1mL) Filtered Centrifuged | 171 nm |

| | | | | | | |
|---|---|---|---|---|---|---|
| Ex.: Examples *OM: Oleylic magnetite, FOM: fluorescent magnetite | | | | | | |

### [Example 20] Evaluation of fluorescent magnetite-enclosed particulate Preparation Uptake by Hepatic cells when administered to Mouse

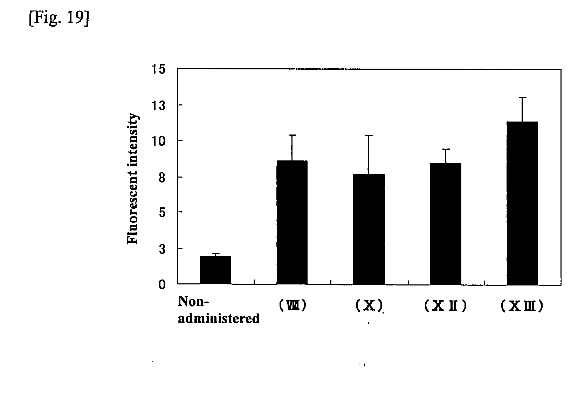

The uptake of fluorescent magnetite-enclosed particulate preparations, which are prepared using amphiphilic compounds (VIII), (X), (XII), and (XIII), by hepatic parenchymal cells was evaluated in accordance with the method of Reference Example 11. In all of them, the uptake of particles by hepatic parenchymal cells was approved. Evaluated results are shown in Fig. 19.

### [Example 21] MRI contrast effect by PLGA matrix particle enclosing magnetite and containing Amphiphilic Compound (VIII) as constituent element

In order to confirm the contrast effect of the particle prepared in Example 6, a magnetic resonance imaging (MRI) was carried out (Visart 1.5 Tesla manufactured by TOSHIBA). A model mouse with metastatic liver cancer (BALB/c, male) obtained by implanting a mouse colon cancer cell colon 26 to the liver was used as a subject for the imaging. First, the imaging was carried out for the mouse before administering the magnetite particle, to obtain a T2 weighted image. After the imaging, the magnetite particle solution (applied dose: 0.45 mg/kg as iron amount) suspended in physiological saline was injected via tail vein of the mouse in a single dose, and then MRI was carried out from 5 minutes after the injection. As a result of the imaging, the MR signal decay from before to after the injection at the liver part is clearly observed, and accordingly the contrast effect on the liver was shown. In addition, the MR signal decay in a metastatic cancer of nodal part was not observed, and thus an obvious contrast against the normal liver part was recognized. Accordingly, it was realized that the detection of a metastatic liver cancer is available by using the magnetite particle. The imaging was carried out chronologically over 4 hours after the injection. It was confirmed that there is almost no difference in the contrast effect from 5 minutes after the injection to 4 hours after the injection, and there is a sufficient contrast effect at the point of 5 minutes after the injection.

### [Example 22] MRI contrast effect by PLGA matrix particle enclosing magnetite and containing Amphiphilic Compound (XIII) as constituent element

In order to confirm the contrast effect of the particle prepared in Example 18, a magnetic resonance imaging (MRI) was carried out (Visart 1.5 Tesla manufactured by TOSHIBA). A rat (Wistar, ♂) was used as a subject for the imaging. First, the imaging was carried out for the rat before administering the magnetite particle, to obtain a T2 weighted image. After the imaging, the magnetite particle solution (applied dose: 0.45mg/kg as iron amount) suspended in physiological saline was injected via tail vein of the rat in a single dose, and then MRI was carried out from 30 minutes after the injection. As a result of the imaging, the MR signal decay from before to after the injection at the liver part is clearly observed, and accordingly the contrast effect on the liver is shown. From the obtained image, the signal intensity of a liver part and a muscle part was determined. When a signal intensity ratio between organs calculated in accordance with the following expression was compared in terms of before and after the injection, it was realized that the signal intensity ratio for the after the injection is significantly decreased by 40% in approximate as compared to the ratio before the injection.

Signal intensity ratio = signal intensity of liver before (or after) injection/signal intensity of muscle before (or after) injection

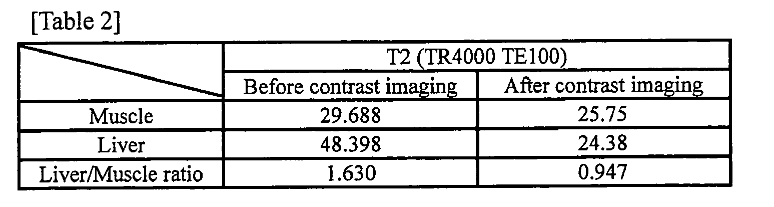

### INDUSTRIAL APPLICABILITY

The pharmaceutical preparation of the invention has no protein adsorbability, thus is hardly taken up by the reticuloendothelial system and forms a particulate pharmaceutical preparation which well retains in blood.

When the pharmaceutical preparation of the invention is used for delivering various drugs, a prolonged pharmacological effect is expected. In addition, when the ligand for delivering purpose is selected for the pharmaceutical preparation of the invention, a biodegradable amphiphilic compound which has no branched chains thus easy to be synthesized and a cluster of ligands such as sugars on a particle surface can be formed. Thus, a particulate preparation exhibiting a remarkable site targeting property can be formed. Accordingly, selective delivery of drugs to only specified organs or disordered site becomes possible, and compared to a systemic administration, the efficacy due to the increased amount of drug delivered to a target site is increased or the total administered amount of drug for giving same effect is reduced, thus the side effect is also reduced.

Moreover, according to the selection of matrix forming material for the pharmaceutical preparation of the invention, the preparation of particle having high drug retentivity can be also applied to various drugs, and according to the selection of biodegradability and solubility of matrix forming material, the drug releasing property can be controlled.

Further, when a diagnostic drug such as an MRI contrast agent is used as a drug in the pharmaceutical preparation of the invention, a site-specific MRI contrast becomes possible, and high definition and highly accurate diagnosis of diseased site by MRI can be achieved as compared to the known contrast agents.

## Claims

1. A pharmaceutical preparation comprising an amphiphilic compound represented by the following general formula (I): 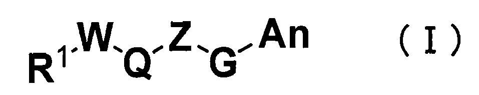 wherein R¹ is a ligand structure which specifically recognizes a target site; W is a group which includes any one of -NH-, -O-, and -S-; Q is a chained hydrophilic group; Z is any of -O-, -S-, and -NR² (where R² is hydrogen, a methyl group, an ethyl group, a normal propyl group, an isopropyl group, an acetyl group, a benzyl group, a hydroxy group, or a methoxy group); G is a group represented by the following general formula (II): 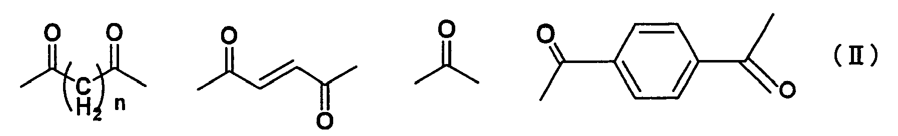 (where n is an integer of 0 to 9); and An is a hydrophobic or amphiphilic group.

2. The pharmaceutical preparation according to claim 1, wherein Q in the general formula (I) is any one of polyethyleneglycol, polyethyleneimine, polyoxyethylene oxide, peptide, nucleic acid, and a derivative residue thereof.

3. The pharmaceutical preparation according to claim 1, wherein An in the general formula (I) is represented by the following formula (III) and/or formula (IV): 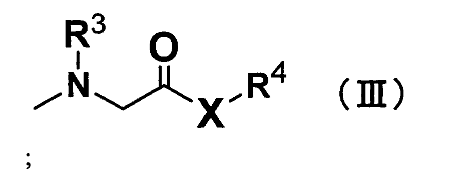 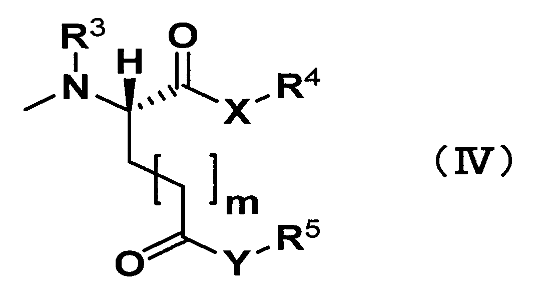 wherein R³ is any one of hydrogen, a methyl group, an ethyl group, a normal propyl group, an isopropyl group, an acetyl group, a benzyl group, a hydroxy group, and a methoxy group; X and Y are each independently or same -NR⁶-, -O-, or -S-, (where R⁶ is hydrogen or an alkyl group having 1 to 20 carbon atom(s)); R⁴ and R⁵ are each hydrogen or an alkyl group having 1 to 20 carbon atom(s); and m is an integer of 0 to 4.

4. The pharmaceutical preparation according to claim 3, wherein R⁴ and R⁵ are each a hydrophobic or amphiphilic group represented by a straight-chained alkyl group having 1 to 20 carbon atom(s), a branched alkyl group having 1 to 20 carbon atom(s), a straight-chained alkyl group having 2 to 20 carbon atoms which contains a double bond, a branched alkyl group having 2 to 20 carbon atoms which contains a double bond, or -CH₂R⁷ (where R⁷ is an aryl group or a cycloalkyl group having 3 to 8 carbon atoms).

5. The pharmaceutical preparation according to claim 3, wherein R⁶ is a hydrophobic or amphiphilic group represented by a straight-chained alkyl group having 1 to 20 carbon atom(s), a branched alkyl group having 1 to 20 carbon atom(s), a straight-chained alkyl group having 2 to 20 carbon atoms which contains a double bond, a branched alkyl group having 2 to 20 carbon atoms which contains a double bond, or -CH₂R⁷ (where R⁷ is an aryl group or a cycloalkyl group having 3 to 8 carbon atoms).

6. The pharmaceutical preparation according to claim 1, wherein R¹ in the general formula (I) is any one of a monosaccharide and/or a derivative thereof, a disaccharide and/or a derivative thereof, an oligosaccharide and/or a derivative thereof, and a polysaccharide and/or a derivative thereof.

7. The pharmaceutical preparation according to claim 1, wherein R¹ in the general formula (I) is any one of a monosaccharide, a disaccharide, and an oligosaccharide, and the end saccharide of the monosaccharide, the disaccharide, and the oligosaccharide is any of galactose, N-acetylgalactosamine, mannose, glucose, N-acetylglucosamine, and maltose.

8. The pharmaceutical preparation according to claim 1, wherein R¹ in the general formula (I) is any one of trisaccharide 2'-fucosyllactose, tetrasaccharide 2',3-difucosyllactose, trisaccharide 2,3-difucosyllactose, and an aldonic acid derivative thereof.

9. The pharmaceutical preparation according to claim 1, wherein R¹ in the general formula (I) is any one of a Lewis X-type trisaccharide chain, a sialyl Lewis X-type tetrasaccharide chain, a 3'-sialyl lactosamine trisaccharide chain, a 6'-sialyl lactosamine trisaccharide chain, and an aldonic acid derivative thereof.

10. The pharmaceutical preparation according to claim 1, wherein Q in the general formula (I) is a chained hydrophilic group represented by the following general formula (V):

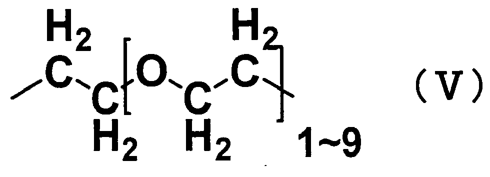

11. The pharmaceutical preparation according to claim 1, wherein W in the general formula (I) is a group represented by the following general formula (VI): 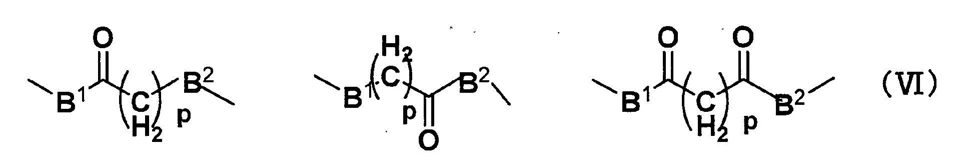 wherein B1 and B2 are independently or same -O-, -NH-, or -S-; and p is an integer of 0 to 9.

12. The pharmaceutical preparation according to claim 1, further comprising a matrix forming material.

13. The pharmaceutical preparation according to claim 12, wherein the matrix forming material is a biodegradable polymer, oils and fats, a liposome forming material, or/and perfluorocarbon.

14. The pharmaceutical preparation according to claim 13, wherein the biodegradable polymer is any of aliphatic polyester, polyanhydride, polycarbonate, polyorthoester, poly-alpha-cyanoacrylic acid ester, polyphosphazene, polypeptide, and polyamino acid.

15. The pharmaceutical preparation according to claim 1, wherein the pharmaceutical preparation is formed with a particle having the average particle size of 50 µm or less.

16. The pharmaceutical preparation according to claim 1, wherein the pharmaceutical preparation is formed with a particle having the average particle size of 10 to 300 nm.

17. The pharmaceutical preparation according to claim 1, further comprising a drug.

18. The pharmaceutical preparation according to claim 17, wherein the drug is a diagnostic drug or a therapeutic drug for liver diseases.

19. The pharmaceutical preparation according to claim 17, wherein the drug is any of an antineoplastic drug, an antimicrobial drug, an antiviral drug, an antiinflammatory drug, and a diagnostic drug.

20. The pharmaceutical preparation according to claim 19, wherein the diagnostic drug is an MRI contrast agent.

21. The pharmaceutical preparation according to claim 20, wherein the MRI contrast agent is a supermagnetic metal oxide or/and a paramagnetic metal complex.

22. A pharmaceutical preparation comprising hydrophobic supermagnetic metal oxide, a biodegradable polymer, an amphiphilic compound, and a particle which has an average particle size of 25 to 300 nm.

23. The pharmaceutical preparation according to claim 22, wherein the biodegradable polymer is any of fatty acid polyester, polyanhydride, polycarbonate, polyorthoester, poly-alpha-cyanoacrylic acid ester, polyphosphazene, polypeptide, and polyamino acid.

24. The pharmaceutical preparation according to claim 22, wherein the hydrophobic supermagnetic metal oxide is supermagnetic metal oxide to which fatty acid or the salt thereof is bonded.

25. The pharmaceutical preparation according to claim 23, wherein the amphiphilic compound is lipid or surfactant.

26. The pharmaceutical preparation according to claim 22, wherein the amphiphilic compound is an amphiphilic compound having peptide, protein, or a saccharide segment, in the structure.

27. The pharmaceutical preparation according to claim 22, wherein the amphiphilic compound is an amphiphilic polymer.
